# EUROPEAN PATENT APPLICATION

(11) **EP 4 596 668 A1**
(43) Date of publication of application: **06.08.2025**
(21) Application number: 22959937.8
(22) Date of filing: 28.09.2022
(51) Int. Cl.: C12M 1/36, C12M 1/34, G01N 33/48

(54) **CHIP PROCESSING DEVICE, GENE SEQUENCER, AND METHOD FOR PERFORMING BIOCHEMICAL DETECTION**

(71) Applicant: MGI Tech Co., Ltd., Shenzhen, Guangdong 518083 (CN)
(72) Inventor: CHEN, Zehua, Shenzhen, Guangdong 518083 (CN); LU, Hao, Shenzhen, Guangdong 518083 (CN); WANG, Jia, Shenzhen, Guangdong 518083 (CN); SUN, Leilin, Shenzhen, Guangdong 518083 (CN); XING, Chutian, Shenzhen, Guangdong 518083 (CN)
(74) Representative: Haseltine Lake Kempner LLP
(86) International application number: PCT/CN2022/122191
(87) International publication number: WO 2024/065292

(57) **Abstract**

Provided are a chip processing device integrated with a reagent kit, a gene sequencer, a gene sequencing apparatus, and a method of performing biochemical detection. The chip processing device includes a substrate extending in a first direction, and a reagent kit platform and a chip platform assembled side-by-side and adjacently on the substrate in a second direction transverse to the first direction. The chip platform has a chip receiving area for accommodating a chip, the reagent kit platform has an accommodating chamber, and the reagent kit is received in the accommodating chamber. The reagent kit has a first fluid transport structure, the chip platform has a second fluid transport structure located on the chip platform and at least partially overlap and communicate with the first fluid transport structure. The first fluid transport structure is in fluid communication with the chip via the second fluid transport structure.

## Description

### TECHNICAL FIELD

The present disclosure relates to the field of sequencing technology, and in particular, to a chip processing device integrated with a reagent kit, a gene sequencer, a gene sequencing apparatus, and a method of performing biochemical detection.

### BACKGROUND

In existing chip platforms for introducing a reagent into a chip for biochemical reactions or optical photography imaging, such as in a gene sequencer, the reagent is usually guided, using a pipe joint, to the chip for biochemical reactions or optical photography, and a reagent kit and a reagent needle are independent of the chip platform and connected through the pipe joint; an existing valve component is usually independent of a chip installation platform and connected to the chip installation platform through the pipe joint; fluid path connections between various functional modules generate various pipelines, and the existing chip installation platform and reagent kit platform are two independent platforms, not an integrated assembly part or combination. In the process of gene sequencing, each cycle requires a biochemical reaction. Generally, the reagent required for the biochemical reaction is supplied by the reagent needle provided on the reagent kit. Specifically, the biochemical reaction may be carried out by using an injection pump and a selector valve such as a rotary valve to pump the reagent from the corresponding reagent needle into the chip.

Moreover, the separate reagent kit, the reagent needle, and the selector valve are usually discretely provided outside the chip platform, and such discrete arrangement of various components leads to lower integration and space utilization, and may correspondingly lead to a complex fluid passage arrangement in which a fluid passage length is difficult to reduce. Specifically, the reagent kit usually serves to accommodate and supply different reagents, the reagent needle serves to insert into the reagent kit and export different reagents from the reagent kit, and the selector valve usually serves as a key component in the fluid path arrangement, functioning to control fluid paths of different reagents and cleaning solutions. By switching different fluid paths, the fluid path between the reagent kit and the chip is connected through the selector valve, thereby selectively achieving entry and exit of the reagent and the cleaning solution. Such conventional arrangement with a large number of discrete components typically require numerous pipe joints and manifold fluid paths, and a rational layout of the manifold fluid paths within a limited installation space is required so as to avoid mutual crossing and minimize mutual routing interference, and docking, switching, and sealing between valve ports and outlets of the manifold fluid paths at the selector valve are also required to be considered during design. Moreover, existing discrete component designs require a large reagent kit stroke and a long fluid path within a limited space, i.e., require large stroke horizontal displacement and lifting so as to achieve transport of fluids, such as the reagent, from the reagent needle to the sequencing chip. In addition, there are conflicting requirements for switching between different fluid paths and cleaning of residual fluids between each switching, as well as for secure fixation and fine leveling of the reagent kit and the chip, and for precise positioning of the reagent kit.

### SUMMARY

In order to solve at least one aspect of the above-mentioned problems and defects in the prior art, an objective of the present disclosure is to provide a chip processing device integrated with a reagent kit, a gene sequencer, a gene sequencing apparatus, and a method of applying a gene sequencer.

In order to achieve the above-mentioned objective, the technical solution of the present disclosure is implemented through the following methods.

In a first aspect of the present disclosure, a chip processing device integrated with a reagent kit is provided, comprising: a substrate extending in a first direction, and a reagent kit platform and a chip platform assembled side-by-side and adjacently on the substrate in a second direction transverse to the first direction, wherein a top plate of the chip platform on a side away from the substrate has a chip receiving area for accommodating a chip carrying a sample for fluid detection, the reagent kit platform is formed with a hollow accommodating chamber, and the reagent kit is received in the accommodating chamber. The reagent kit has a first fluid transport structure located inside the reagent kit on a side towards the chip platform in the second direction, the chip platform has a second fluid transport structure located on a side of the chip platform towards the reagent kit platform in the second direction and configured to at least partially overlap and communicate with the first fluid transport structure in a case that the reagent kit platform is assembled with the chip platform, and the first fluid transport structure is in fluid communication with the chip via the second fluid transport structure.

In embodiments of the present disclosure, the accommodating chamber is coupled to the substrate in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively; and wherein the chip processing device is configured to: in response to the accommodating chamber being lifted away from the substrate to the fluid guiding position, the first fluid transport structure is engaged with the second fluid transport structure for fluid communication; and in response to the accommodating chamber being lowered towards the substrate to the non-fluid guiding position, the first fluid transport structure is separated from the second fluid transport structure.

In embodiments of the present disclosure, the top plate on a side of the chip platform away from the substrate has a flange protruding towards the reagent kit platform in the second direction, and the reagent kit platform is partially embedded and assembled between the flange of the top plate and the substrate.

In embodiments of the present disclosure, the first fluid transport structure comprises a plurality of reagent slots and a plurality of guide slots arranged in one-to-one correspondence inside the reagent kit, and a communication channel in fluid communication between a bottom of each of the plurality of guide slots and a bottom of a corresponding reagent slot, each reagent slot is at least partially filled with a fluid and has a first opening open upward towards the flange and a first pierceable structure covering the first opening, and each guide slot has a second opening open upward towards the flange and a second pierceable structure covering the second opening.

In embodiments of the present disclosure, the second fluid transport structure comprises a fluid supply device, and the fluid supply device comprises: a fluid path network formed in the top plate of the chip platform and communicated between the first fluid transport structure and the chip, and a selector valve installed to the top plate and in fluid communication with the fluid path network.

In embodiments of the present disclosure, the selector valve comprises: a valve seat, wherein the selector valve is fixed to the top plate via the valve seat; and a valve body, extending from the valve seat in a direction away from the top plate, wherein the valve body is formed with a fluid inlet configured to guide the fluid to flow into an interior of the valve body and a fluid outlet configured to guide the fluid to flow outward from the interior of the valve body, wherein the fluid path network comprises: a plurality of fluid guiding needle ports located on a side of the top plate opposite to the chip receiving area; a plurality of inlet ports spaced apart from the plurality of fluid guiding needle ports in one-to-one correspondence; a plurality of branch fluid passages, each of the plurality of branch fluid passages is communicated between each fluid guiding needle port and a corresponding inlet port, and is configured to guide the fluid input from each of the plurality of fluid guiding needle port to the corresponding inlet port; an outlet port spaced apart from the plurality of inlet ports and not in communication with the plurality of branch fluid passages; and a common fluid passage communicated between the outlet port and the chip receiving area, the common fluid passage is configured to guide the fluid output from the outlet port to the chip receiving area, and wherein the selector valve is arranged such that the fluid outlet is in fluid communication with the outlet port, and the fluid inlet is in fluid communication with at least one of the plurality of inlet ports, and the selector valve is configured to switch a selective communication between at least one of the plurality of inlet ports corresponding to the plurality of branch fluid passages and the outlet port via the fluid inlet and the fluid outlet.

In embodiments of the present disclosure, the plurality of fluid guiding needle ports are penetratively formed in a protruding portion at an edge of the top plate and protruding towards a side of the top plate away from the chip receiving area.

In embodiments of the present disclosure, the second fluid transport structure further comprises a fluid guiding component protruding from the flange towards the reagent kit platform and being communicated to the chip receiving area, and the fluid guiding component comprises: a plurality of membrane breaking needles and a plurality of fluid guiding needles respectively protruding from a side of the top plate opposite to the chip receiving area towards the reagent kit platform, each of the plurality of membrane breaking needles has a first end aligned with a corresponding first pierceable structure, and each of the plurality of fluid guiding needles has a second end aligned with a corresponding second pierceable structure, wherein each membrane breaking needle is not connected to the fluid path network and is configured to, in response to the accommodating chamber reaching the fluid guiding position, pierce the first pierceable structure with the first end and then insert into the reagent slot to expose the reagent slot so as to change an air pressure inside the reagent slot; and wherein each fluid guiding needle is configured as a hollow needle, in fluid communication with a corresponding branch fluid passage of the plurality of branch fluid passages in one-to-one correspondence, and is configured to, in response to a situation where the accommodating chamber reaches the fluid guiding position, pierce the second pierceable structure with the second end and then insert into the guide slot for fluid communication to the guide slot so as to draw the fluid in the guide slot.

In embodiments of the present disclosure, in a case that the plurality of fluid guiding needles are inserted into the guide slots, a free end of each of the plurality of fluid guiding needles is higher than an inner wall at a bottom of the reagent slot.

In embodiments of the present disclosure, each guide slot has a slot having a circular cross-section with a first inner diameter, and each guide slot has a slot having a circular cross-section with a second inner diameter smaller than the first inner diameter.

In embodiments of the present disclosure, the first pierceable structure is a metal foil; and wherein the second pierceable structure is a flexible sealing element, such that a liquid-tight seal is maintained after being pierced by a corresponding fluid guiding needle of the plurality of fluid guiding needles.

In embodiments of the present disclosure, the plurality of fluid guiding needles are spaced apart from each other in a straight line, and the plurality of membrane breaking needles are spaced apart from each other in a straight line; and wherein the plurality of fluid guiding needles and the plurality of membrane breaking needles are arranged parallel to each other.

In embodiments of the present disclosure, a corresponding membrane breaking needle is provided aside each fluid guiding needle in one-to-one correspondence, each fluid guiding needle is in pair with the corresponding single membrane breaking needle, and each fluid guiding needle and the corresponding single membrane breaking needle are arranged adjacent to and spaced apart from each other.

In embodiments of the present disclosure, each fluid guiding needle comprises a hollow and elongated straight tubular needle body, and a through terminal with a tapered longitudinal cross-section.

In embodiments of the present disclosure, the plurality of fluid guiding needles are installed to the plurality of fluid guiding needle ports in a threaded connection manner.

In embodiments of the present disclosure, the fluid guiding component further comprises: at least two guide pins protruding outward from a side of the top plate opposite to the chip receiving area and arranged spaced apart from each other in a straight line, and configured to be in positive fit with alignment features on the reagent kit.

In embodiments of the present disclosure, the plurality of fluid guiding needle ports are aligned in a straight line.

In embodiments of the present disclosure, the plurality of fluid guiding needle ports are spaced from each other by a uniform spacing.

In embodiments of the present disclosure, the selector valve is a rotary valve configured to be rotatable about an axis of the rotary valve to switch a selective communication between at least one of the plurality of inlet ports corresponding to the plurality of branch fluid passages and the outlet port via the fluid inlet and the fluid outlet.

In embodiments of the present disclosure, an axial direction of the inlet port and an axial direction of the outlet port are parallel to the axis, and the inlet port is offset from the axis, and the outlet port is coaxial with the axis.

In embodiments of the present disclosure, the selector valve is installed to a back side of the top plate away from the chip receiving area via the valve seat, and wherein the plurality of inlet ports are arranged in a circular shape around the axis, the plurality of inlet ports and the outlet port are open to the back side of the top plate, and at least one of the plurality of inlet ports is selectively communicated to the fluid inlet of the valve body, the outlet port is communicated to the fluid outlet of the valve body, and a center-to-center distance between each of the plurality of inlet ports and the outlet port is equal.

In embodiments of the present disclosure, the plurality of branch fluid passages diverge radially around the rotary valve to communicate with the plurality of fluid guiding needle ports in one-to-one correspondence, respectively.

In embodiments of the present disclosure, the top plate comprises: a top layer, wherein the chip receiving area is formed in the top layer; and a support layer stacking with the top layer, and the support layer is located between the top layer and the selector valve, and wherein the plurality of branch fluid passages and the common fluid passage are formed on a side of the support layer towards the top layer, and the plurality of inlet ports, the outlet port, and the plurality of fluid guiding needle ports extend to penetrate the support layer.

In embodiments of the present disclosure, the chip receiving area of the top layer is formed therein a through accommodating hole, and a recess recessing from a side of the top layer away from the support layer and arranged around the accommodating hole.

In embodiments of the present disclosure, the top plate further comprises a chip adsorption component partially fixed in the accommodating hole and protruding away from the support layer from the accommodating hole, the chip adsorption component is configured to accommodate and adsorb the chip, and the chip adsorption comprises: a pipe joint in communication with a negative pressure source; an adsorption table partially arranged in the accommodating hole, wherein an edge of a top surface of the adsorption table protrudes outward to define an adsorption slot in the top layer recessing towards the support layer and located between the edge and the top surface, the adsorption slot is configured as a closed slot extending along the edge and in a closed loop form, and a shape and a size of the adsorption slot are determined to be suitable for surrounding and securing the edge of the chip; and a negative pressure channel penetratively formed in the adsorption table and communicated between the pipe joint and the adsorption slot.

In embodiments of the present disclosure, the top layer is formed with a discharge port and a supply port penetrating the top layer and extending to the recess, the supply port is in fluid communication with the outlet port of the valve body via the common fluid passage, and the discharge port is spaced apart from the supply port.

In embodiments of the present disclosure, the common fluid passage is coupled between the outlet port and the supply port in a straight line.

In embodiments of the present disclosure, the plurality of branch fluid passages are arranged not to cross each other and to avoid the common fluid passage.

In embodiments of the present disclosure, end portions of the discharge port and the supply port respectively extend to the recess; and wherein in a case that the chip is received and adsorbed on the adsorption table, the adsorption table and the chip jointly define a first liquid-tight sealing surface, and the chip is in a sealed fluid communication with the supply port and the discharge port respectively at the first liquid-tight sealing surface.

In embodiments of the present disclosure, the support layer is formed with a discharge channel penetrating the support layer and in fluid communication from the discharge port to an outside of the top plate.

In embodiments of the present disclosure, the plurality of inlet ports and the outlet port extend to penetrate the support layer to the back side of the top plate, and the back side and the valve seat jointly define a second liquid-tight sealing surface, and the plurality of inlet ports and the outlet port respectively form a sealed fluid communication with the fluid inlet and the fluid outlet of the valve body at the second liquid-tight sealing surface.

In embodiments of the present disclosure, the chip processing device further comprises a driving component inside, at least one guide rail component coupled between the accommodating chamber and the substrate, the accommodating chamber is movable in a direction orthogonal to the substrate via a transmission of at least one guide rail component by the driving component, so that the fluid guiding component is inserted into and fluidly communicated to the reagent kit.

In embodiments of the present disclosure, the driving component comprises: a driving source comprising one of a stepper motor and a piezoelectric driver; and a lead screw in transmission coupling between the driving source and at least one guide rail component.

In embodiments of the present disclosure, each guide rail component comprises two spaced sets of cross roller guide rails, each set of cross roller guide rails comprises a fixed rail fixed to the chip platform, a movable rail coupled to the accommodating chamber, and a plurality of rolling elements held between the fixed rail and the movable rail.

In embodiments of the present disclosure, in each set of cross roller guide rails, the fixed rail is fixed to the chip platform in a manner orthogonal to the substrate, and the movable rail is fixed to the accommodating chamber in a manner orthogonal to the substrate.

In embodiments of the present disclosure, the lead screw is coupled with the respective movable rail of the two sets of cross roller guide rails in at least one guide rail component.

In embodiments of the present disclosure, the chip processing device further includes a positioning device, wherein the positioning device comprises: a groove concavely formed in a top-side inner wall of the accommodating chamber; an elastic component arranged in the groove, and a positioning bead arranged at an end of the elastic component towards the substrate and configured to: in response to a situation where the reagent kit does not reach the positioning bead within the accommodating chamber, the elastic component is in an initial state not subjected to a force applied by the reagent kit, and the positioning bead protrudes at least partially towards the substrate from the top-side inner wall of the accommodating chamber; and in response to a situation where the reagent kit is inserted into the accommodating chamber and the positioning bead is pressed, the elastic component is pushed towards the groove via the positioning bead, thereby causing the elastic component to retract at least partially into the groove.

In embodiments of the present disclosure, the reagent kit has a protruding portion protruding towards the top-side inner wall of the accommodating chamber, and a positioning groove recessing from the protruding portion, and in response to a situation where the reagent kit is inserted into the accommodating chamber and the positioning bead is pressed, the positioning bead is clamped between the positioning groove and the elastic component.

In embodiments of the present disclosure, the chip processing device further includes an in-position detector inside the accommodating chamber, wherein the in-position detector comprises an optocoupler component and a shielding member, the optocoupler component comprises an infrared emitter and an infrared receiver and is configured to determine that the reagent kit is in position inside the accommodating chamber, in response to the shielding member being displaced or deformed by a force applied by the reagent kit and reception of infrared rays from the infrared emitter by the infrared receiver being blocked when the reagent kit is inserted in position inside the accommodating chamber.

In embodiments of the present disclosure, the chip processing device further comprises a pressing device formed at a top of the reagent kit platform, wherein the top of the reagent kit platform is formed with a through opening to at least partially expose the reagent kit inserted into the accommodating chamber, and the pressing device comprises: a platen, wherein one side of the platen is fixed to the reagent kit platform; a pin penetrating a free side of the platen opposite to the one side; a pair of arms, wherein one end of each of the pair of arms is rotatably coupled to a corresponding end of two ends of the pin; and a pair of elastic members, wherein each elastic member is elastically coupled between a corresponding arm of the pair of arms and the reagent kit platform.

In embodiments of the present disclosure, the chip processing device further includes a first tilt adjustment mechanism arranged between a bottom of the reagent kit platform and the substrate, wherein the first tilt adjustment mechanism comprises a first fix distance head and two first thread pairs being rotatable and adjustable relative to the substrate that are arranged in a non-straight line.

In embodiments of the present disclosure, the chip processing device further includes a second tilt adjustment mechanism arranged between a bottom of the chip platform and the substrate, wherein the second tilt adjustment mechanism comprises a second fix distance head and two second threaded pairs being rotatable and adjustable relative to the substrate that are arranged in a non-straight line.

In embodiments of the present disclosure, the chip processing device further includes a position limiting device, including: an induction sheet arranged on a corresponding movable rail of at least one set of cross roller guide rails in at least one guide rail component; and an upper position limiting optocoupler and a lower position limiting optocoupler respectively arranged at positions corresponding to upper and lower ends of a stroke of the corresponding movable rail at two ends of the corresponding fixed rail of the at least one set of cross roller guide rails, and respectively configured to stop the driving source in response to determining that the movable rail is lifted to to an upper limit position by detecting that the upper position limiting optocoupler is blocked by the induction sheet, and to stop the driving source in response to determining that the movable rail is lowered to a lower limit position by detecting that the lower position limiting optocoupler is blocked by the induction sheet.

In embodiments of the present disclosure, the chip processing device further includes a power component in fluid communication with the fluid path network, wherein the power component is configured to drive the fluid to flow through the fluid path network towards the supply port.

In embodiments of the present disclosure, the power component comprises a pump in communication with the chip receiving area, and the pump is configured to provide a negative pressure to the chip receiving area.

In embodiments of the present disclosure, the chip processing device further includes a temperature control component arranged on a side of the top plate opposite to the chip receiving area and configured to regulate a temperature of the fluid supplied from the fluid guiding component to the chip receiving area.

In embodiments of the present disclosure, the temperature control component comprises a first temperature adjustment device arranged adjacent to the selector valve on a same side of the top plate, and a fluid guiding device further comprises an adapter bracket fixed to a side of the top plate opposite to the chip receiving area and configured as a form of a frame, and the adapter bracket is formed with two concave cavities arranged side-by-side and recessing from opposite sides of the adapter bracket away from the top plate and towards the top plate, respectively, so as to respectively accommodate and fix the selector valve and the first temperature adjustment device inside.

In embodiments of the present disclosure, the first temperature adjustment device comprises: a heat dissipation component accommodated and installed in one of the two concave cavities of the adapter bracket recessing from a side towards the top plate, wherein the heat dissipation component comprises: at least one of an active heat dissipation component and a passive heat dissipation component; and a temperature control module installed to the heat dissipation component and configured to cut off the power component when a temperature at the heat dissipation component exceeds a threshold temperature.

In embodiments of the present disclosure, the temperature control component further comprises a heat conducting member inserted between the top plate and the first temperature adjustment device.

In embodiments of the present disclosure, the heat conducting member comprises a phase change material.

In embodiments of the present disclosure, the active heat dissipation component comprises a thermoelectric cooler; or the passive heat dissipation component comprises one of: a single heat sink or a heat sink array.

In embodiments of the present disclosure, the temperature control component further comprises a second temperature adjustment device fixed to a side of the adapter bracket away from the top plate and arranged side-by-side with the selector valve, and the second temperature adjustment device is aligned with the first temperature adjustment device.

In embodiments of the present disclosure, the second temperature adjustment device comprises a fan, and the fan comprises: a hollow first housing defining a cavity inside for air flow, wherein the first housing is arranged such that the cavity is aligned with the first temperature adjustment device and open towards the first temperature adjustment device, so as to fluidly communicate the cavity between the first temperature adjustment device and an outside of the fluid guiding device; and a fan component accommodated inside the first housing, wherein the fan component comprises: a second housing configured as a hollow cylindrical body fixedly sleeved inside the first housing; a rotating shaft rotatably installed inside the second housing; and a plurality of fan blades coaxially fixed to the rotating shaft inside the second housing and rotatable with the rotating shaft relative to the second housing.

In embodiments of the present disclosure, the adapter bracket is further formed with a gas channel penetrating an inside of the adapter bracket and open at opposite ends respectively towards cavities of the first temperature adjustment device and the second temperature adjustment device, and the cavities are communicated to the first temperature adjustment device via the gas channel.

In embodiments of the present disclosure, the second temperature adjustment device further comprises a vibration reduction device, and the vibration reduction device comprises: a first level vibration reduction structure configured as a gasket inserted between the adapter bracket and the first housing of the fan; and a second level vibration reduction structure arranged inside the first housing of the fan between the second housing of the fan component and the first housing and, wherein the second level vibration reduction structure comprises a plurality of vibration reduction members respectively snap-fitted to an inner wall of the first housing and spaced apart from each other, and the second housing of the fan component is coupled to the first housing via the plurality of vibration reduction members.

In embodiments of the present disclosure, at least one of the first level vibration reduction structure and the second level vibration reduction structure is a vibration compensation device, and the vibration compensation device is an elastic member or a damping member.

In embodiments of the present disclosure, the first housing of the fan is fixed to the adapter bracket via a threaded member of a flexible vibration absorbing material, and the first level vibration reduction structure is pressed between the adapter bracket and the first housing via the threaded member.

In embodiments of the present disclosure, the selector valve is fixed to the top plate via a threaded connection at the valve seat, and the selector valve is coupled to the adapter bracket via an adjustable abutting device, and the adjustable abutting device comprises: a plurality of fix distance screws respectively threadably penetrating the adapter bracket and abutting against the valve body of the selector valve; and a plurality of springs elastically abutting against the plurality of fix distance screws towards the top plate in one-to-one correspondence, respectively.

According to a second aspect of the present disclosure, a gene sequencer is provided, including: a chip carrying a sample for fluid detection; and a chip processing device comprising a substrate extending in a first direction, and a reagent kit platform and a chip platform assembled side-by-side and adjacently on the substrate in a second direction transverse to the first direction, wherein a top plate of the chip platform on a side away from the substrate has a chip receiving area for accommodating the chip, the reagent kit platform is formed with a hollow accommodating chamber, and the reagent kit is received in the accommodating chamber and at least partially filled with a fluid inside. The reagent kit has a first fluid transport structure located inside the reagent kit on a side towards the chip platform in the second direction, the chip platform has a second fluid transport structure located on a side of the chip platform towards the reagent kit platform in the second direction and configured to at least partially overlap and communicate with the first fluid transport structure in a case that the reagent kit platform is assembled with the chip platform; The reagent kit is removably inserted into the accommodating chamber, and the fluid in the reagent kit is in fluid communication with the sample carried on the chip via the first fluid transport structure and the second fluid transport structure.

In embodiments of the present disclosure, the accommodating chamber is coupled to the substrate in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively; and wherein the chip processing device is configured to: in response to the accommodating chamber being lifted away from the substrate to the fluid guiding position, the first fluid transport structure is engaged with the second fluid transport structure for fluid communication; and in response to the accommodating chamber being lowered towards the substrate to the non-fluid guiding position, the first fluid transport structure is separated from the second fluid transport structure.

In embodiments of the present disclosure, the top plate on a side of the chip platform away from the substrate has a flange protruding towards the reagent kit platform in the second direction, and the reagent kit platform is partially embedded and assembled between the flange of the top plate and the substrate.

In embodiments of the present disclosure, the first fluid transport structure comprises a plurality of reagent slots and a plurality of guide slots arranged in one-to-one correspondence inside the reagent kit, and a communication channel in fluid communication between a bottom of each guide slot and a bottom of a corresponding reagent slot, each reagent slot is at least partially filled with the fluid and has a first opening open upward towards the flange and a first pierceable structure covering the first opening, and each guide slot has a second opening open upward towards the flange and a second pierceable structure covering the second opening.

In embodiments of the present disclosure, the second fluid transport structure comprises a fluid supply device, and the fluid supply device comprises: a fluid path network formed in the top plate of the chip platform and communicated between the first fluid transport structure and the chip, and a selector valve installed to the top plate and in fluid communication with the fluid path network.

In embodiments of the present disclosure, the selector valve comprises: a valve seat, wherein the selector valve is fixed to the top plate via the valve seat; and a valve body, extending from the valve seat in a direction away from the top plate, wherein the valve body is formed with a fluid inlet configured to guide the fluid to flow into an interior of the valve body and a fluid outlet configured to guide the fluid to flow outward from the interior of the valve body, wherein the fluid path network comprises: a plurality of fluid guiding needle ports located on a side of the top plate opposite to the chip receiving area; a plurality of inlet ports spaced apart from the plurality of fluid guiding needle ports in one-to-one correspondence; a plurality of branch fluid passages, each of the plurality of branch fluid passages is communicated between each fluid guiding needle port and a corresponding inlet port, and is configured to guide the fluid input from each of the plurality of fluid guiding needle port to the corresponding inlet port; an outlet port spaced apart from the plurality of inlet ports and not in communication with the plurality of branch fluid passages; and a common fluid passage communicated between the outlet port and the chip receiving area, the common fluid passage is configured to guide the fluid output from the outlet port to the chip receiving area, and wherein the selector valve is arranged such that the fluid outlet is in fluid communication with the outlet port, and the fluid inlet is in fluid communication with at least one of the plurality of inlet ports, and the selector valve is configured to switch a selective communication between at least one of the plurality of inlet ports corresponding to the plurality of branch fluid passages and the outlet port via the fluid inlet and the fluid outlet.

In embodiments of the present disclosure, the plurality of fluid guiding needle ports are penetratively formed in a protruding portion at an edge of the top plate and protruding towards a side of the top plate away from the chip receiving area.

In embodiments of the present disclosure, the second fluid transport structure further comprises a fluid guiding component protruding from the flange towards the reagent kit platform and being communicated to the chip receiving area, and the fluid guiding component comprises: a plurality of membrane breaking needles and a plurality of fluid guiding needles respectively protruding from a side of the top plate opposite to the chip receiving area towards the reagent kit platform, each of the plurality of membrane breaking needles has a first end aligned with a corresponding first pierceable structure, and each of the plurality of fluid guiding needles has a second end aligned with a corresponding second pierceable structure, wherein each membrane breaking needle is not connected to the fluid path network and is configured to, in response to the accommodating chamber reaching the fluid guiding position, pierce the first pierceable structure with the first end and then insert into the reagent slot to expose the reagent slot so as to change an air pressure inside the reagent slot; and wherein each fluid guiding needle is configured as a hollow needle, in fluid communication with a corresponding branch fluid passage of the plurality of branch fluid passages in one-to-one correspondence, and is configured to, in response to a situation where the accommodating chamber reaches the fluid guiding position, pierce the second pierceable structure with the second end and then insert into the guide slot for fluid communication to the guide slot so as to draw the fluid in the guide slot.

In embodiments of the present disclosure, in a case that the plurality of fluid guiding needles are inserted into the guide slots, a free end of each of the plurality of fluid guiding needles is higher than an inner wall at a bottom of the reagent slot.

In embodiments of the present disclosure, each guide slot has a slot having a circular cross-section with a first inner diameter, and each guide slot has a slot having a circular cross-section with a second inner diameter smaller than the first inner diameter.

In embodiments of the present disclosure, the first pierceable structure is a metal foil; and wherein the second pierceable structure is a flexible sealing element, such that a liquid-tight seal is maintained after being pierced by a corresponding fluid guiding needle of the plurality of fluid guiding needles.

According to a third aspect of the present disclosure, a gene sequencing apparatus is provided, including: a chip carrying a sample for fluid detection; and at least two chip processing devices, wherein each of the at least two chip processing devices comprises a substrate extending in a first direction, and a reagent kit platform and a chip platform assembled side-by-side and adjacently on the substrate in a second direction transverse to the first direction, wherein a top plate of the chip platform on a side away from the substrate has a chip receiving area for accommodating the chip, the reagent kit platform is formed with a hollow accommodating chamber, and the reagent kit is received in the accommodating chamber and at least partially filled with a fluid inside. The reagent kit has a first fluid transport structure located inside the reagent kit on a side towards the chip platform in the second direction, the chip platform has a second fluid transport structure located on a side of the chip platform towards the reagent kit platform in the second direction and configured to at least partially overlap and communicate with the first fluid transport structure in a case that the reagent kit platform is assembled with the chip platform. The reagent kit is removably inserted into the accommodating chamber, and the fluid in the reagent kit is in fluid communication with the sample carried on the chip via the first fluid transport structure and the second fluid transport structure. The at least two chip processing devices are arranged symmetrically with respect to each other, and the respective chip platforms are arranged adjacent to each other.

In embodiments of the present disclosure, the at least two chip processing devices comprise at least one pair of chip processing devices arranged in mirror symmetry with the respective chip platforms arranged adjacent to each other.

In embodiments of the present disclosure, the accommodating chamber is coupled to the substrate in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively; and wherein the chip processing device is configured to: in response to the accommodating chamber being lifted away from the substrate to the fluid guiding position, the first fluid transport structure is engaged with the second fluid transport structure for fluid communication; and in response to the accommodating chamber being lowered towards the substrate to the non-fluid guiding position, the first fluid transport structure is separated from the second fluid transport structure.

In embodiments of the present disclosure, the top plate on a side of the chip platform away from the substrate has a flange protruding towards the reagent kit platform in the second direction, and the reagent kit platform is partially embedded and assembled between the flange of the top plate and the substrate.

In embodiments of the present disclosure, the first fluid transport structure comprises a plurality of reagent slots and a plurality of guide slots arranged in one-to-one correspondence inside the reagent kit, and a communication channel in fluid communication between a bottom of each guide slot and a bottom of a corresponding reagent slot, each reagent slot is at least partially filled with the fluid and has a first opening open upward towards the flange and a first pierceable structure covering the first opening, and each guide slot has a second opening open upward towards the flange and a second pierceable structure covering the second opening.

In embodiments of the present disclosure, the second fluid transport structure comprises a fluid supply device, and the fluid supply device comprises: a fluid path network formed in the top plate of the chip platform and communicated between the first fluid transport structure and the chip, and a selector valve installed to the top plate and in fluid communication with the fluid path network.

In embodiments of the present disclosure, the selector valve comprises: a valve seat, wherein the selector valve is fixed to the top plate via the valve seat; and a valve body, extending from the valve seat in a direction away from the top plate, wherein the valve body is formed with a fluid inlet configured to guide the fluid to flow into an interior of the valve body and a fluid outlet configured to guide the fluid to flow outward from the interior of the valve body, wherein the fluid path network comprises: a plurality of fluid guiding needle ports located on a side of the top plate opposite to the chip receiving area; a plurality of inlet ports spaced apart from the plurality of fluid guiding needle ports in one-to-one correspondence; a plurality of branch fluid passages, each of the plurality of branch fluid passages is communicated between each fluid guiding needle port and a corresponding inlet port, and is configured to guide the fluid input from each of the plurality of fluid guiding needle port to the corresponding inlet port; an outlet port spaced apart from the plurality of inlet ports and not in communication with the plurality of branch fluid passages; and a common fluid passage communicated between the outlet port and the chip receiving area, the common fluid passage is configured to guide the fluid output from the outlet port to the chip receiving area, and wherein the selector valve is arranged such that the fluid outlet is in fluid communication with the outlet port, and the fluid inlet is in fluid communication with at least one of the plurality of inlet ports, and the selector valve is configured to switch a selective communication between at least one of the plurality of inlet ports corresponding to the plurality of branch fluid passages and the outlet port via the fluid inlet and the fluid outlet.

In embodiments of the present disclosure, the plurality of fluid guiding needle ports are penetratively formed in a protruding portion at an edge of the top plate and protruding towards a side of the top plate away from the chip receiving area.

In embodiments of the present disclosure, the second fluid transport structure further comprises a fluid guiding component protruding from the flange towards the reagent kit platform and being communicated to the chip receiving area, and the fluid guiding component comprises: a plurality of membrane breaking needles and a plurality of fluid guiding needles respectively protruding from a side of the top plate opposite to the chip receiving area towards the reagent kit platform, each of the plurality of membrane breaking needles has a first end aligned with a corresponding first pierceable structure, and each of the plurality of fluid guiding needles has a second end aligned with a corresponding second pierceable structure, wherein each membrane breaking needle is not connected to the fluid path network and is configured to, in response to the accommodating chamber reaching the fluid guiding position, pierce the first pierceable structure with the first end and then insert into the reagent slot to expose the reagent slot so as to change an air pressure inside the reagent slot; and wherein each fluid guiding needle is configured as a hollow needle, in fluid communication with a corresponding branch fluid passage of the plurality of branch fluid passages in one-to-one correspondence, and is configured to, in response to a situation where the accommodating chamber reaches the fluid guiding position, pierce the second pierceable structure with the second end and then insert into the guide slot for fluid communication to the guide slot so as to draw the fluid in the guide slot.

In embodiments of the present disclosure, in a case that the plurality of fluid guiding needles are inserted into the guide slots, a free end of each of the plurality of fluid guiding needles is higher than an inner wall at a bottom of the reagent slot.

In embodiments of the present disclosure, each guide slot has a slot having a circular cross-section with a first inner diameter, and each guide slot has a slot having a circular cross-section with a second inner diameter smaller than the first inner diameter.

In embodiments of the present disclosure, the first pierceable structure is a metal foil; and wherein the second pierceable structure is a flexible sealing element, such that a liquid-tight seal is maintained after being pierced by a corresponding fluid guiding needle of the plurality of fluid guiding needles.

According to a fourth aspect of the present disclosure, a method of performing biochemical detection is provided, including: establishing a fluid connection between a chip with a sample for fluid detection and a reagent kit with a plurality of different reaction components, wherein the reaction components comprise at least one of a specimen generation component or a specimen analysis component; optionally, generating a specimen on the chip in a generation operation, wherein the generation operation comprises flowing different specimen generation components into the chip and controlling reaction conditions of the chip to generate the specimen; and analyzing the specimen of the chip in an analysis operation, wherein the analysis operation comprises flowing the specimen analysis component into the chip, and the specimen analysis component reacts with the specimen to provide a relevant detectable signal. A reagent kit and a chip are integrated into a chip processing device, and a fluid in the reagent kit is in fluid communication with the chip via a first fluid transport structure and a second fluid transport structure separated from each other in the chip processing device.

In embodiments of the present disclosure, the first fluid transport structure is integrated into the reagent kit, the second fluid transport structure is integrated into a carrier supporting the chip, and the carrier is integrated into the chip processing device.

In embodiments of the present disclosure, the reagent kit moves relative to the carrier through a movable bracket integrated into the chip processing device so as to achieve a communication between the first fluid transport structure and the second fluid transport structure.

In embodiments of the present disclosure, the biochemical reaction is a nucleic acid sequencing reaction, and the specimen to be detected is a nucleic acid sequencing library.

In embodiments of the present disclosure, the specimen to be detected is a tissue specimen, and the biochemical reaction is a specific binding reaction.

In embodiments of the present disclosure, the detectable signal is an optical signal.

In embodiments of the present disclosure, the communication between the first fluid transport structure and the second fluid transport structure comprises: the first fluid transport structure is communicated with an external air pressure of the chip processing device to drive the fluid in the reagent kit to flow into the second flow transport structure; and the chip is selectively communicated with the fluid in the second flow transport structure.

In embodiments of the present disclosure, the second flow transport structure has a plurality of branch fluid passages, and the selective communication between the chip and the fluid comprises: controlling selective communications between different branch fluid passages and the chip using a selector valve integrated in the chip processing device.

The technical solution provided in the present disclosure has the following advantages: the chip processing device, the gene sequencer, the gene sequencing apparatus, and the method of using the gene sequencer implemented in embodiments of the present disclosure, especially the chip processing device, may achieve an integrated ductless fluid transport structure through the above arrangements, which has a reduced fluid passage length, more reliable leveling and fastening effects and higher positioning accuracy for the reagent kit. Furthermore, the arrangement of sucking liquid from the top of the reagent kit in combination with the use of the injection pump may avoid the long stroke translating and lifting movements and also avoid the need to insert the reagent needle into the bottom of the reagent kit as in conventional operations. Accordingly, it is possible to improve the fixation, positioning, stroke, and other aspects of the fluid transport structure while enhancing the degree of integration and thereby improving the space utilization rate, and the design expectation is achieved with a more compact structure. Moreover, this compact structure minimizes the space occupation, and the simple construction and coupling relationship facilitate assembly and disassembly.

### BRIEF DESCRIPTION OF THE DRAWINGS

FIG. 1(a) schematically shows a perspective structural view of a chip processing device according to an embodiment of the present disclosure. FIG. 1(b) is an exploded schematic diagram of FIG. 1(a). FIG. 1(c) shows a top view of a part of the structure of FIG. 1(a). FIG. 1(d) is a schematic sectional view taken along line P-P in FIG. 1(c). FIG. 1(e) is a schematic sectional view of the structure shown in FIG. 1(e) at the same position after removing a reagent chamber 110. FIG. 1(f) is a perspective schematic diagram of the structure shown in FIG. 1(a) after removing a reagent chamber 110.

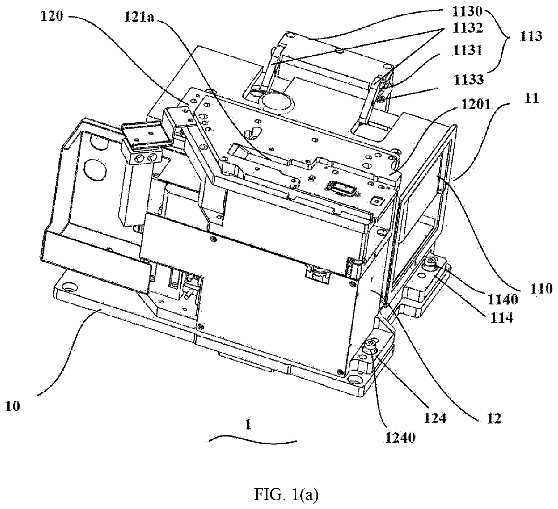

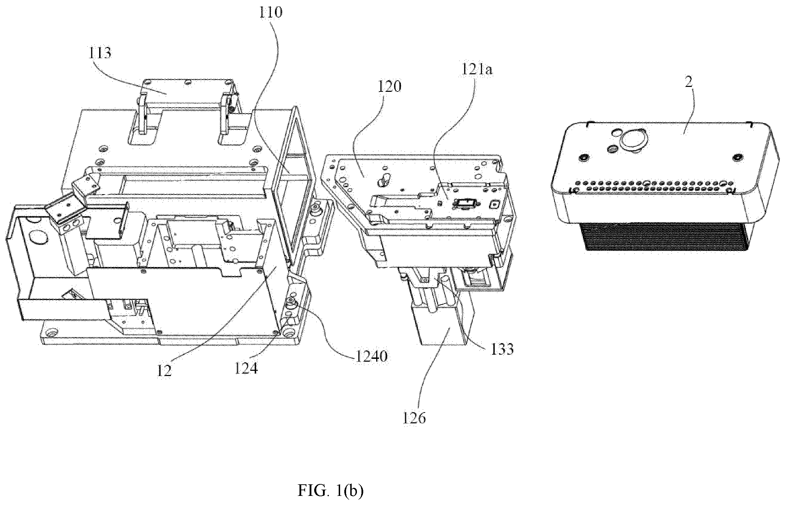

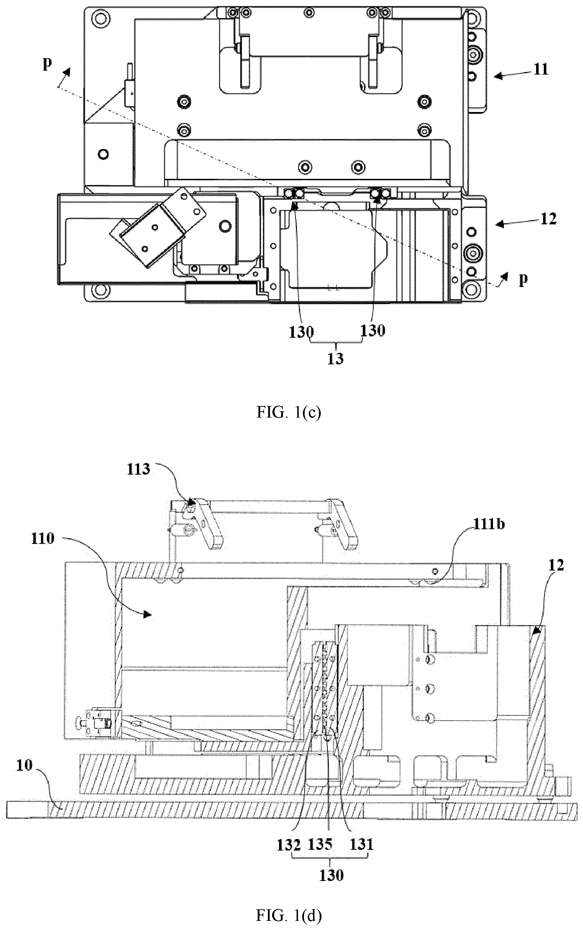

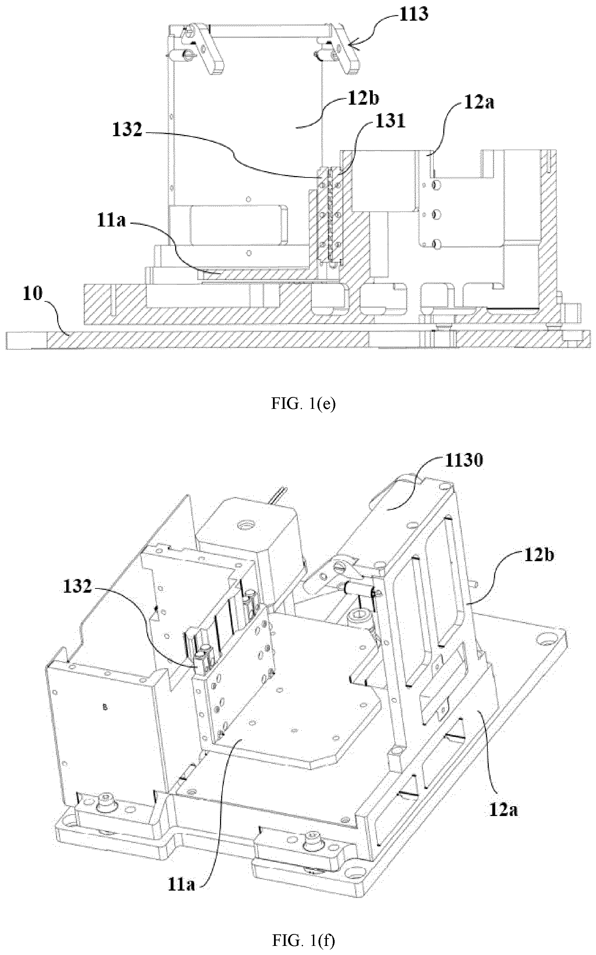

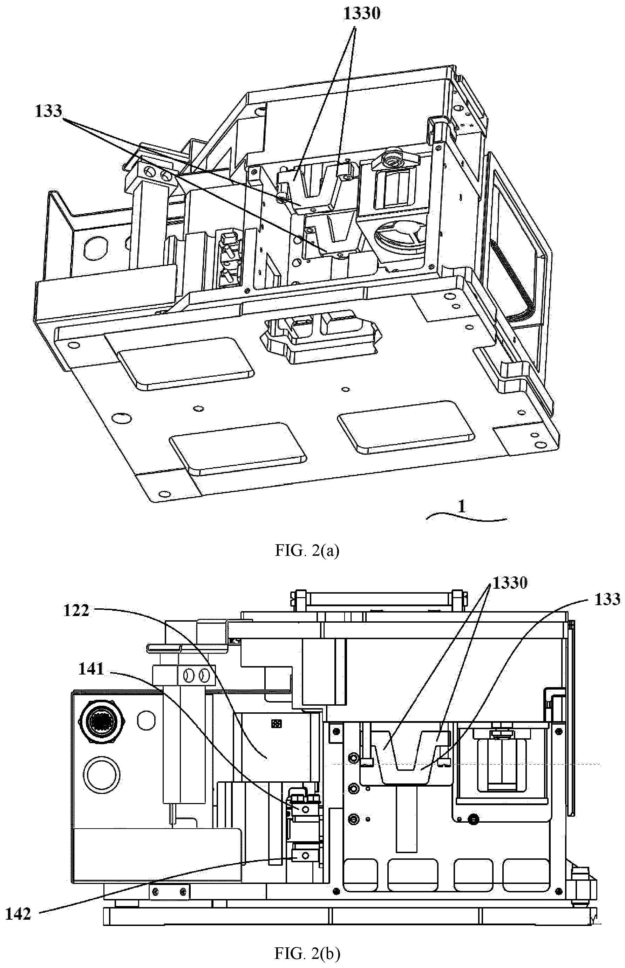

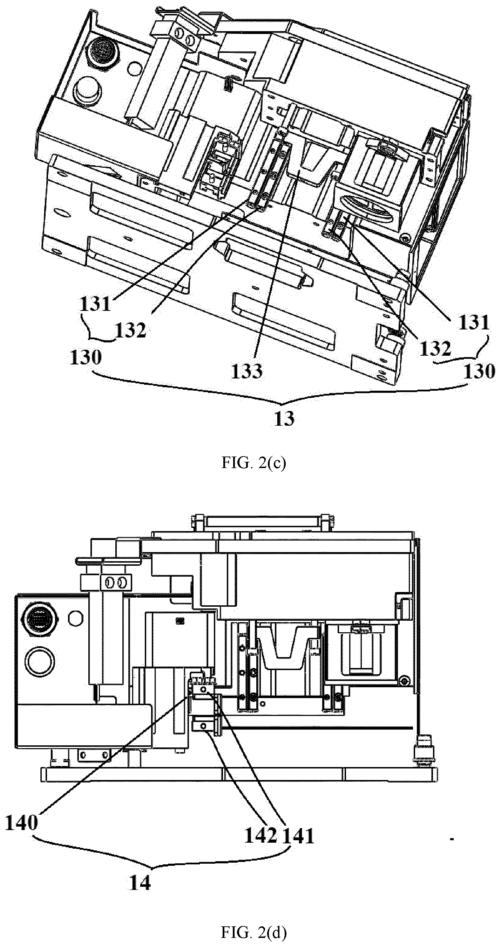

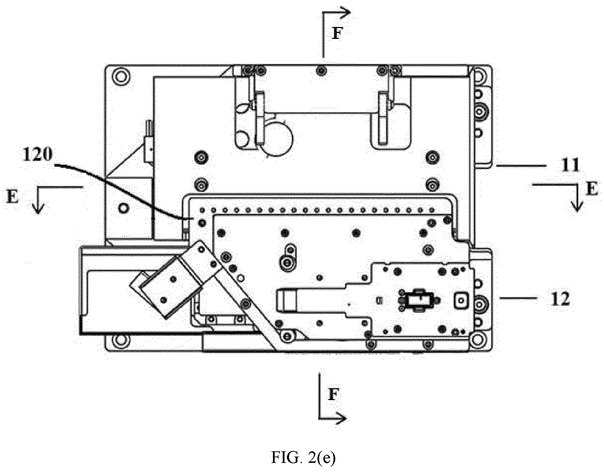

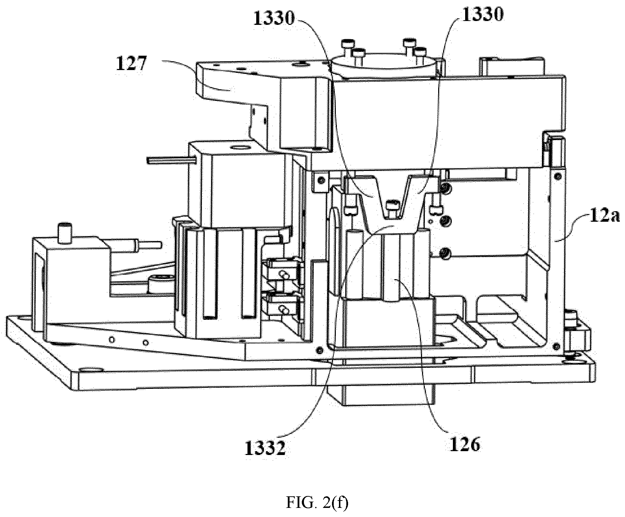

FIG. 2(a) to FIG. 2(e) schematically show views from different angles of the chip processing device shown in FIG. 1. FIG. 2(a) is a schematic perspective view, in which a panel of a chip platform and an internal cross roller guide rail are removed. FIG. 2(b) is a front view in which the panel of the chip platform and the internal cross roller guide rail are removed. FIG. 2(c) is a schematic perspective view from another angle, in which the panel of the chip platform as well as an internal V-shaped support arranged outside are removed. FIG. 2(d) is a front view with the panel of the chip platform. FIG. 2(e) is a top view. FIG. 2(f) schematically shows an assembly schematic diagram of a selector valve and an adapter bracket.
FIG. 3(a) shows a schematic structural diagram of a reagent kit as a liquid reservoir. FIG. 3(b) shows a top view of the reagent kit of FIG. 3(a), in which a top panel is removed.
FIG. 4 shows a sectional view taken along line F-F in FIG. 2(e), in which a specific arrangement of a reagent slot and a guide slot inside a reagent kit is illustrated.
FIG. 5(a) schematically shows a specific structure for transporting a fluid in a top plate of the chip platform in the chip processing device of FIG. 1, serving as a fluid supply device. FIG. 5(b) schematically shows an assembly relationship between the top plate serving as a carrier and a selector valve.
FIG. 6(a) shows a schematic perspective view of a carrier, including a chip adsorption component installed in a chip receiving area. FIG. 6(b) to FIG. 6(c) schematically show exploded views of the carrier of FIG. 6(a) from top and bottom views, respectively, in which the chip adsorption component is removed for simplicity.
FIG. 7 shows a schematic structural diagram of the chip adsorption component.
FIG. 8 schematically shows a sectional view of a top plate serving as a carrier taken along a common fluid passage.
FIG. 9(a) to FIG. 9(f) respectively show a fluid guiding device, particularly a fluid guiding component thereof, in the chip platform of the chip processing device of FIG. 1 from different angles. FIG. 9(a) and FIG. 9(b) are schematic perspective views of the fluid guiding device shown from different angles, respectively. FIG. 9(c) to FIG. 9(f) are a front view, a right view, a rear view, and a top view of the fluid guiding device, respectively.
FIG. 10 shows a sectional view of the chip processing device shown in the top view of FIG. 2(e) taken along line E-E, in which an internal positioning device and an in-position detector used to detect insertion in-position of a liquid reservoir within an accommodating chamber are illustrated.
FIG. 11 is an exploded schematic diagram of FIG. 9(b).
FIG. 12 is a schematic sectional view taken along line A-A in FIG. 9(f).
FIG. 13(a) is an exploded schematic diagram of a second temperature adjustment device in FIG. 11.
FIG. 13(b) to FIG. 13(c) respectively show schematic perspective views observed after cutting along lines C-C and D-D in FIG. 9(f).
FIG. 14 schematically shows a gene sequencer according to an embodiment of the present disclosure, including the chip processing device shown in FIG. 1, a liquid reservoir that is removably inserted into an accommodating chamber of a reagent kit platform in the chip processing device, and a sequencing chip that is removably inserted into a chip receiving area in a top plate of the chip platform.
FIG. 15 shows a gene sequencing apparatus according to an embodiment of the present disclosure, including a pair of aforementioned gene sequencers arranged in mirror symmetry and adjacent to each other.
FIG. 16 shows a schematic flowchart of a biochemical detection method using the gene sequencer shown in FIG. 14 according to an embodiment of the present disclosure.
FIG. 17 schematically shows more detailed steps in the method of the flowchart shown in FIG. 16.

### DETAILED DESCRIPTION OF EMBODIMENTS

Technical solution of the present disclosure will be further described in detail below through embodiments and in combination with the accompanying drawings. In the specification, the same or similar reference signs represent the same or similar components. The following descriptions of embodiments of the present disclosure with reference to the accompanying drawings are intended to explain the general inventive concept of the present disclosure, and should not be construed as limiting the present disclosure. In addition, in the following detailed descriptions, for purposes of explanation, numerous specific details are set forth in order to provide a thorough understanding of embodiments of the present disclosure. However, it is obvious that one or more embodiments may be implemented without these specific details.

FIG. 1(a) schematically shows a perspective structural view of a chip processing device according to an embodiment of the present disclosure. FIG. 1(b) is an exploded schematic diagram of FIG. 1(a). FIG. 1(c) shows a top view of a part of the structure of FIG. 1(a). FIG. 1(d) is a schematic sectional view taken along line P-P in FIG. 1(c).

According to an overall technical concept of embodiments of the present disclosure, for example, as shown in FIG. 1(a) to FIG. 1(d), there is provided a chip processing device 1 integrated with a reagent kit for supplying a fluid to a chip 3 (e.g. a gene sequencing chip 3) from a liquid reservoir 2 (e.g. the reagent kit) including the fluid. The chip processing device 1 includes a substrate 10 extending in a first direction, and a reagent kit platform 11 and a chip platform 12 assembled side-by-side and adjacent to each other on the substrate 10 in a second direction transverse to the first direction. The chip platform 12 has a top plate 120 located at the top thereof, and the top plate 120 serves as a carrier for supporting the chip and carrying a fluid transport structure. More specifically, the top plate 120 on a side of the chip platform 12 away from the substrate has a chip receiving area 121a for accommodating a chip 3 carrying a sample for fluid detection. More specifically, for example, a shape and a size of the chip receiving area 121a are determined to be suitable for accommodating the chip 3, such that the chip 3 is accommodated in the chip receiving area 121a on the top plate 120 of the chip platform 12. The reagent kit platform 11 is formed with a hollow accommodating chamber 110, and the reagent kit is received in the accommodating chamber. For example, the reagent kit platform 11 has the accommodating chamber 110 which is open outwardly (on a lateral side as shown in FIG. 1) and is suitable for receiving a liquid reservoir 2 (such as the reagent kit) including the fluid. Furthermore, as an example, the reagent kit platform has a first fluid transport structure located inside the reagent kit platform on a side towards the chip platform in the second direction. The chip platform has a second fluid transport structure located on a side of the chip platform towards the reagent kit platform in the second direction and configured to at least partially overlap and communicate with the first fluid transport structure in a case that the reagent kit platform is assembled with the chip platform. The first fluid transport structure is in fluid communication with the chip via the second fluid transport structure. As an example, the accommodating chamber is coupled to the substrate in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively. The chip processing device is configured to: in response to the accommodating chamber being lifted away from the substrate to the fluid guiding position, the first fluid transport structure is engaged with the second fluid transport structure for fluid communication; and in response to the accommodating chamber being lowered towards the substrate to the non-fluid guiding position, the first fluid transport structure is separated from the second fluid transport structure. Thus, in the chip processing device integrated with the liftable reagent kit, it is convenient to switch between the fluid communication state and the non-communication state between the first fluid transport structure in the reagent kit platform and the second fluid transport structure in the chip platform.

Moreover, in a specific embodiment, as an example, the top plate on a side of the chip platform away from the substrate has a flange protruding towards the reagent kit platform in the second direction, and the reagent kit platform 11 is partially embedded and assembled between the outwardly protruding flange 1201 of the top plate 120 and the substrate 10.

FIG. 2(a) to FIG. 2(e) schematically show views from different angles of the chip processing device 1 shown in FIG. 1. FIG. 2(a) is a schematic perspective view, in which a panel of the chip platform 12 and an internal cross roller guide rail 130 are removed. FIG. 2(b) is a front view, in which the panel of the chip platform 12 and the internal cross roller guide rail 130 are removed. FIG. 2(c) is a schematic perspective view from another angle, in which the panel of the chip platform 12 as well as the internal V-shaped support 133 arranged outside are removed. FIG. 2(d) is a front view with the panel of the chip platform 12. FIG. 2(e) is a top view.

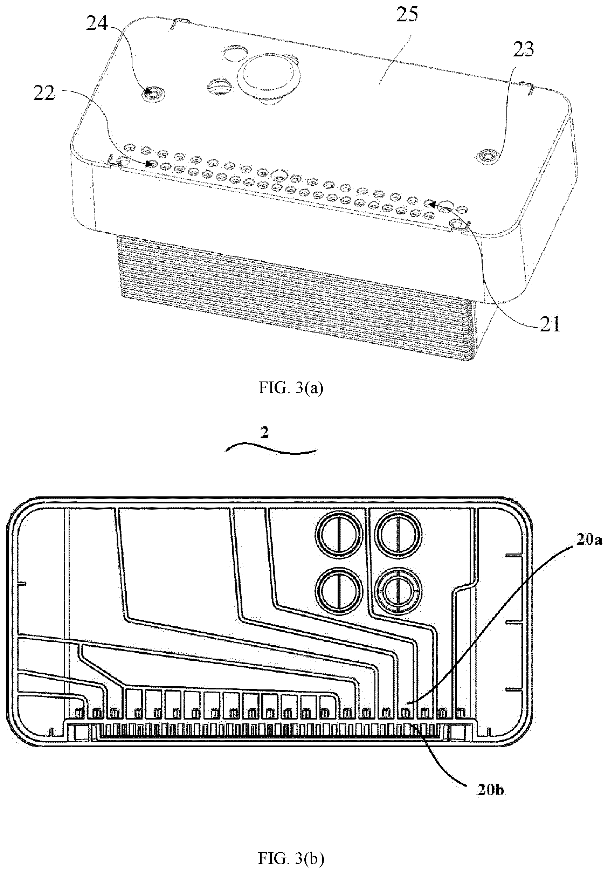

FIG. 3(a) shows a schematic structural diagram of the reagent kit as the liquid reservoir. FIG. 3(b) shows a top view of the reagent kit of FIG. 3(a), in which a top panel is removed.

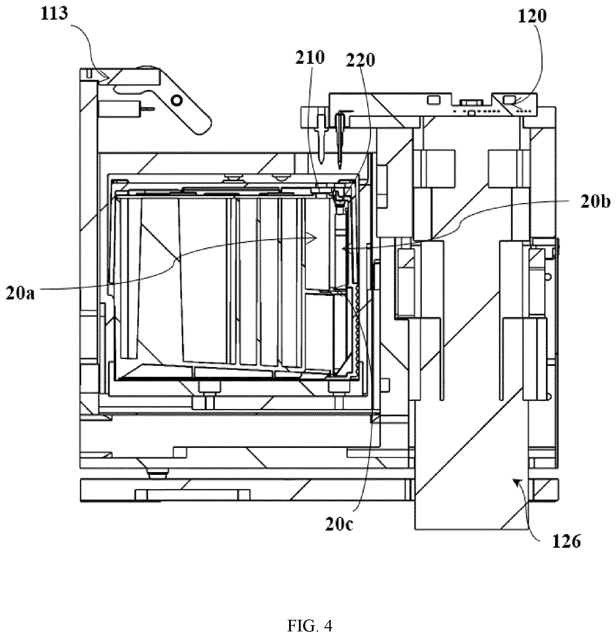

FIG. 4 shows a sectional view taken along line F-F in FIG. 2(e), in which a specific arrangement of a reagent slot and a guide slot inside the reagent kit is illustrated.

In embodiments of the present disclosure, for example, as shown in the figures, the first fluid transport structure includes a plurality of reagent slots 20a and a plurality of guide slots 20b arranged in one-to-one correspondence inside the reagent kit, and a communication channel 20c that is in fluid communication between a bottom of each guide slot 20b and a bottom of a corresponding reagent slot 20a. Each reagent slot 20a is at least partially filled with a fluid and has a first opening 21 open upward towards the flange, and a first pierceable structure 210 covering the first opening 21, and each guide slot 20b has a second opening 22 open upward towards the flange, and a second pierceable structure 220 covering the second opening 22.

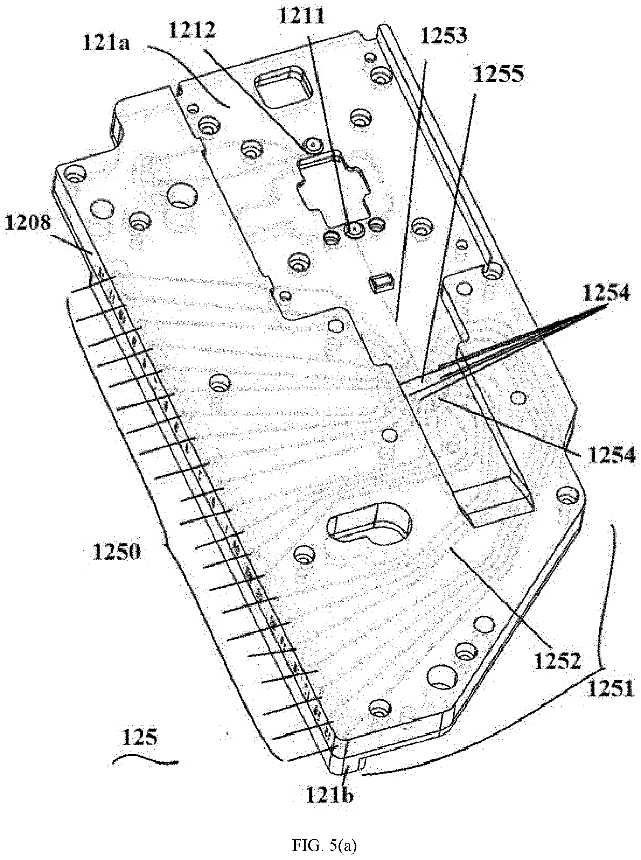

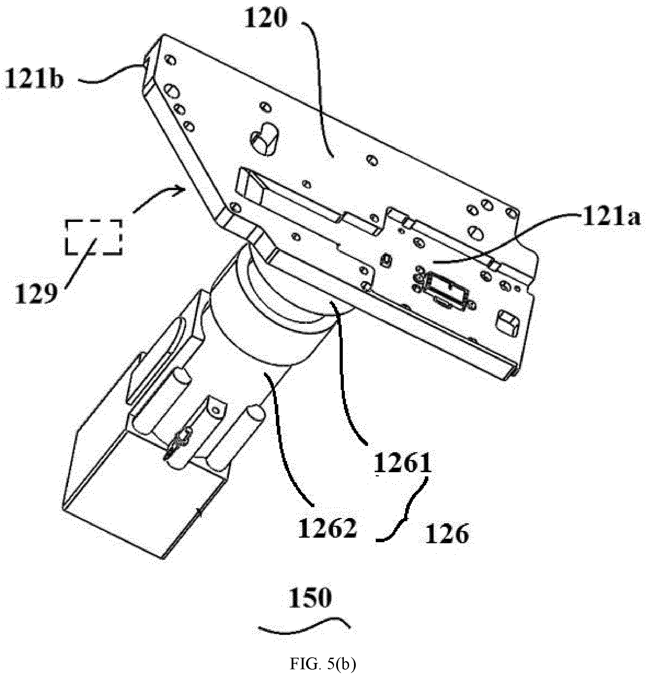

FIG. 5(a) schematically shows a specific structure for transporting the fluid in the top plate of the chip platform in the chip processing device of FIG. 1, serving as a fluid supply device. FIG. 5(b) schematically shows an assembly relationship between the top plate serving as a carrier and a selector valve.

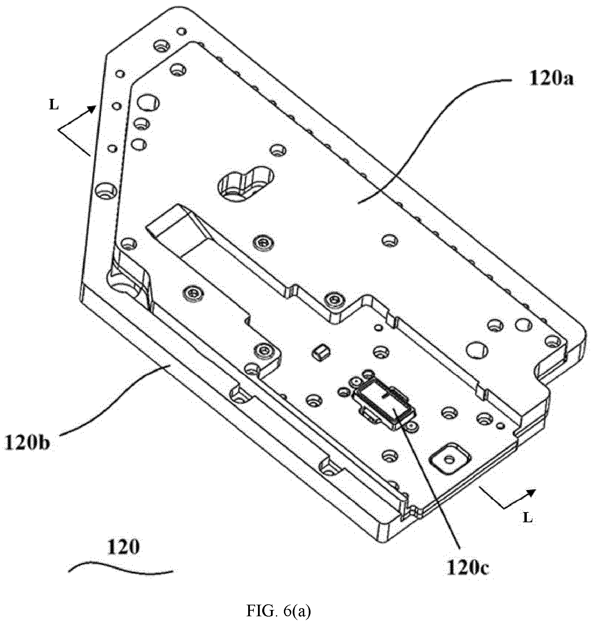

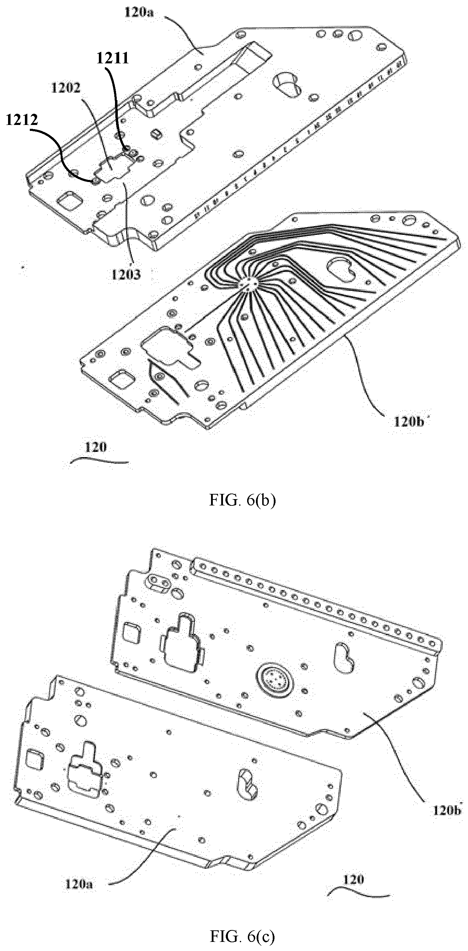

FIG. 6(a) shows a schematic perspective view of the carrier, including a chip adsorption component installed in the chip receiving area. FIG. 6(b) to FIG. 6(c) schematically show exploded views of the carrier of FIG. 6(a) from top and bottom views, respectively, in which the chip adsorption component is removed for simplicity.

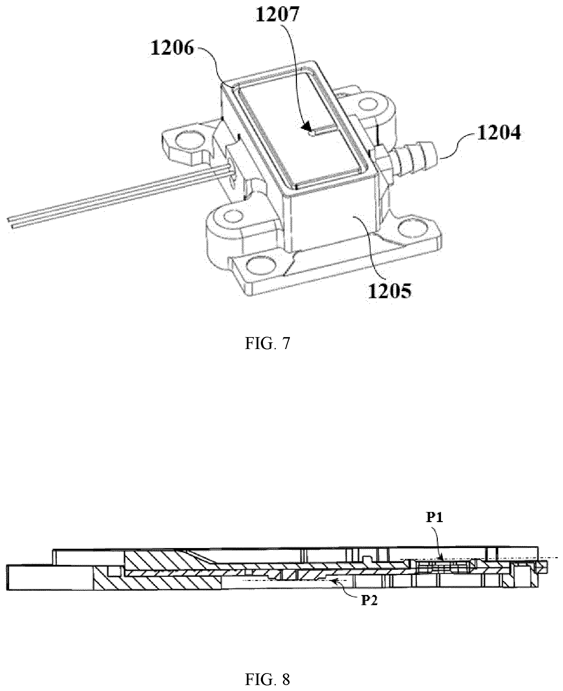

FIG. 7 shows a schematic structural diagram of the chip adsorption component.

FIG. 8 schematically shows a sectional view of the top plate 120 serving as the carrier taken along line L-L where a common fluid passage is located in FIG. 6(a).

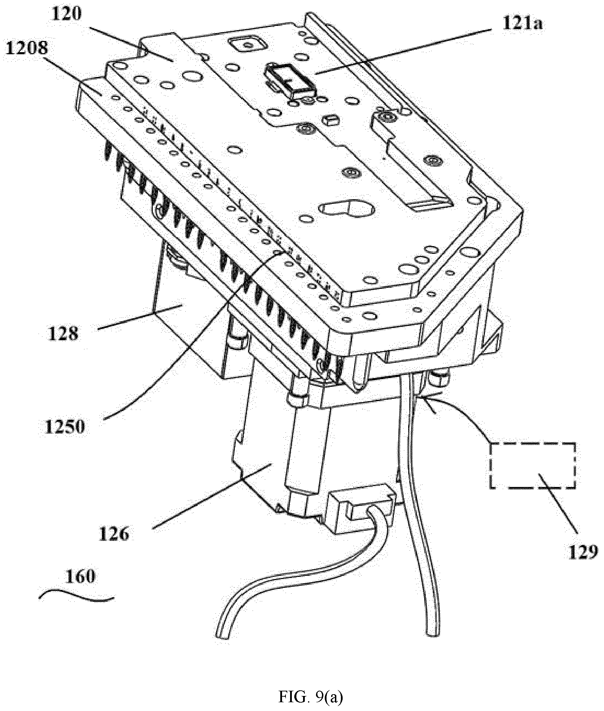

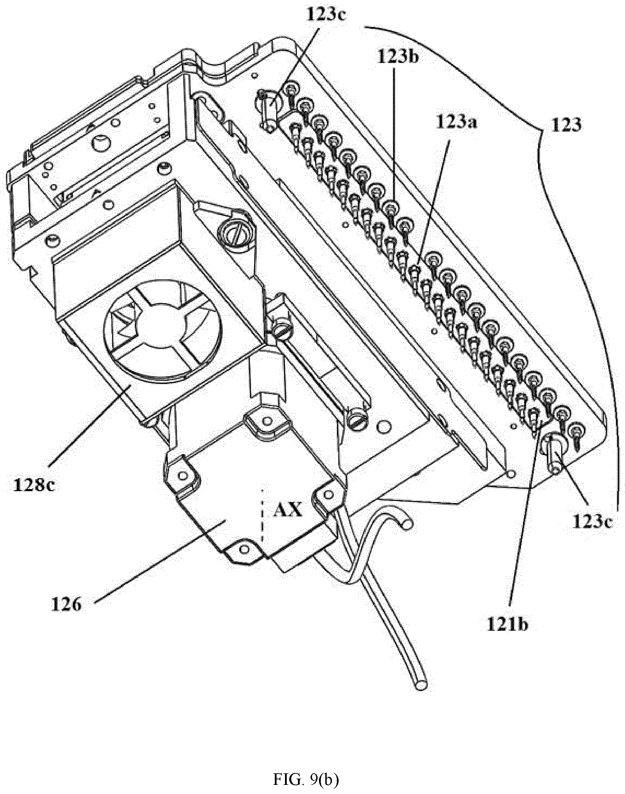

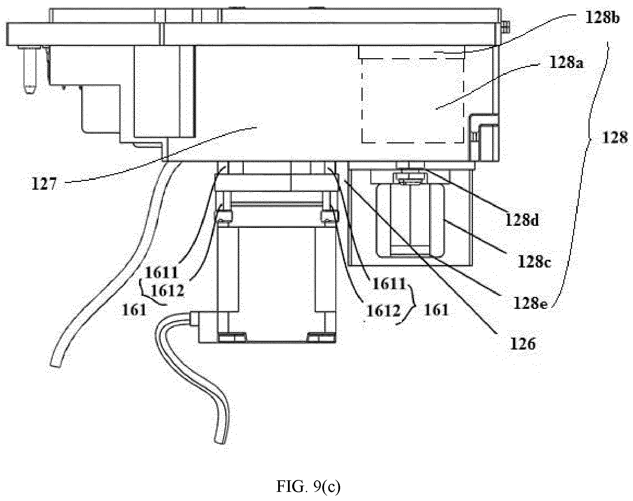

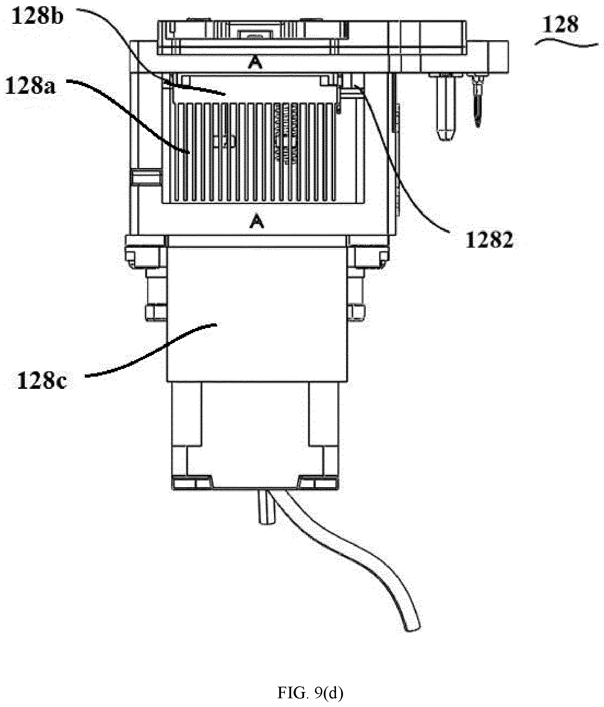

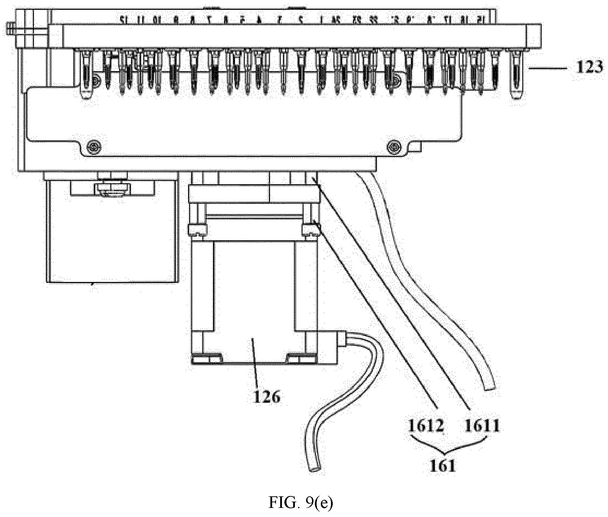

FIG. 9(a) to FIG. 9(f) respectively show a fluid guiding device, particularly a fluid guiding component thereof, in the chip platform of the chip processing device of FIG. 1 from different angles. FIG. 9(a) and FIG. 9(b) are schematic perspective views of the fluid guiding device shown from different angles, respectively. FIG. 9(c) to FIG. 9(f) are a front view, a right view, a rear view, and a top view of the fluid guiding device, respectively.

In a further embodiment, as shown in the figures, the second fluid transport structure includes a fluid supply device. The fluid supply device 150 includes: a fluid path network 125 formed in the top plate of the chip platform and communicated between the first fluid transport structure and the chip, and a selector valve 126 installed to the top plate and in fluid communication with the fluid path network.

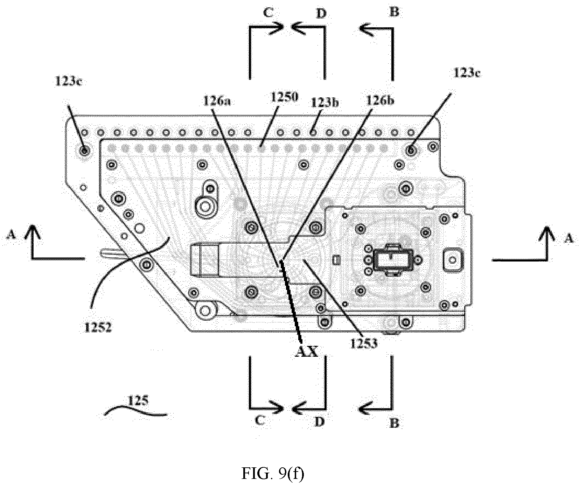

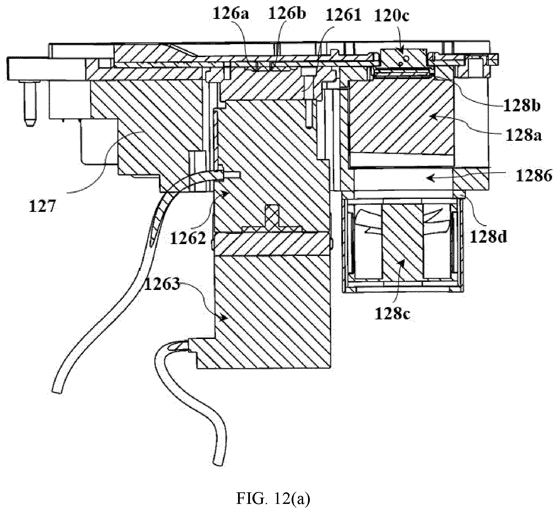

In a further embodiment, as shown in the figures, the selector valve includes a valve seat 1261, the selector valve is fixed to the top plate via the valve seat 1261; and a valve body 1262 extending from the valve seat 1261 in a direction away from the top plate and formed with a fluid inlet 126a configured to guide the fluid to flow into an interior of the valve body 1262 and a fluid outlet 126b configured to guide the fluid to flow outward from the interior of the valve body 1262; and a driving motor 1263 driving at least a rotation of the valve seat so as to achieve communication with different branch fluid passages of the fluid network, the driving motor 1263 is preferably a stepper motor. The fluid path network includes: a plurality of fluid guiding needle ports 1250 located on a side of the top plate opposite to the chip receiving area; a plurality of inlet ports 1254 spaced apart from the plurality of fluid guiding needle ports in one-to-one correspondence; a plurality of branch fluid passages 1252 each being communicated between each fluid guiding needle port and a corresponding inlet port, and being configured to guide the fluid input from each fluid guiding needle port to the corresponding inlet port; an outlet port 1255 spaced apart from the plurality of inlet ports and not communicated with the plurality of branch fluid passages; and a common fluid passage 1253 communicated between the outlet port and the chip receiving area, the common fluid passage is configured to guide the fluid output from the outlet port to the chip receiving area. For example, the selector valve is arranged such that the fluid outlet 126b is in fluid communication with the outlet port, and the fluid inlet 126a is in fluid communication with at least one of the plurality of inlet ports, and the selector valve is configured to switch a selective communication between at least one of the plurality of inlet ports 1254 corresponding to the plurality of branch fluid passages and the outlet port via the fluid inlet 126a and the fluid outlet 126b, as shown in FIG. 9(f) and FIG. 12(a), the fluid inlet 126a represents an interface of the valve body communicated to the upper surface of the valve seat 1261 and selectively communicated with the branch fluid passage of the top plate, and the fluid outlet 126b represents an interface of the valve body communicated to the upper surface of the valve seat 1261 and communicated with the outlet port 1255.

In a further embodiment, for example, the plurality of fluid guiding needle ports are penetratively formed in a protruding portion at an edge of the top plate protruding towards a side of the top plate away from the chip receiving area.

Moreover, as shown in FIG. 1 and FIG. 2(a) to FIG. 2(e), in embodiments according to the present disclosure, for example, in the chip platform 12, the second fluid transport structure further includes a fluid guiding component 123 protruding from the flange 1201 towards the reagent kit platform 11 and being communicated to the chip receiving area 121a. Furthermore, as shown in the figures, the fluid guiding component 123 includes: a plurality of membrane breaking needles 123b and a plurality of fluid guiding needles 123a, respectively protruding from a side of the top plate opposite to the chip receiving area towards the reagent kit platform, each membrane breaking needle has a first end aligned with a corresponding first pierceable structure 210, and each fluid guiding needle has a second end aligned with a corresponding second pierceable structure 220. As an example, each membrane breaking needle is not connected to the fluid path network and is configured to, in response to the accommodating chamber reaching the fluid guiding position, pierce the first pierceable structure 210 with the first end thereof and then insert into the reagent slot 20a to expose the reagent slot 20a so as to change an air pressure inside the reagent slot 20a. As an example, each fluid guiding needle is constructed as a hollow needle, in fluid communication with a corresponding branch fluid passage of the plurality of branch fluid passages in one-to-one correspondence and is configured to, in response to a situation where the accommodating chamber reaches the fluid guiding position, pierce the second pierceable structure 220 with the second end thereof and then insert into the guide slot 20b for fluid communication to the guide slot 20b so as to draw the fluid in the guide slot 20b.

In an exemplary embodiment, for example, in a case that the plurality of fluid guiding needles are inserted into the guide slots 20b, a free end of each of the plurality of fluid guiding needles is higher than an inner wall at a bottom of the reagent slot 20a.

Through the above arrangement, when the reagent kit is lifted to the fluid guiding position along with the accommodating chamber, the membrane breaking needle 123b pierces the first pierceable structure 210 covering the first opening 21 to enter the reagent slot 20a, causing the atmospheric pressure to pass from the pierced first pierceable structure 210 to the reagent slot 20a through the first opening 21, thereby further pressing the reagent into the guide slot 20b via the communication channel 20c at the bottom. At the same time, the fluid guiding needle 123a synchronously pierces the second pierceable structure 220 covering the second opening 22 to enter the guide slot 20b. Through the above-mentioned arrangement, it is convenient for the fluid guiding needle inserted at least partially into the guide slot to achieve suction of the reagent liquid even if the fluid guiding needle is far away from a reagent liquid level in the reagent kit.

In a specific embodiment, as an example, each guide slot 20b is constructed as a slot having a circular cross-section with a first inner diameter, and each guide slot 20b is constructed as a slot having a circular cross-section with a second inner diameter smaller than the first inner diameter. Through such arrangement, it is further convenient for the fluid guiding needle to draw the reagent liquid from the reagent slot into the guide slot via the communication channel at the bottom.

In a specific embodiment, as an example, the first pierceable structure is a metal foil. For example, the second pierceable structure is a flexible sealing element, such that a liquid-tight seal is maintained after being pierced by a corresponding fluid guiding needle of the plurality of fluid guiding needles.

In a specific embodiment, as an example, the plurality of fluid guiding needles are spaced apart from each other in a straight line, and the plurality of membrane breaking needles are spaced apart from each other in a straight line; and the plurality of fluid guiding needles and the plurality of membrane breaking needles are arranged parallel to each other.

In a more specific embodiment, as an example, the plurality of fluid guiding needles 123a are spaced apart from each other at a uniform spacing. Correspondingly, the plurality of reagent dispensing ports are also spaced apart from each other at the spacing. Thereby, an even distribution of the plurality of fluid guiding needles 123a at the aforementioned single edge of the carrier 120 is achieved, effectively avoiding mutual interference and promoting local thermal balance.

Through the above-mentioned arrangement, the plurality of fluid guiding needles 123a in a linear arrangement, distributed at uniform spacing and intensively arranged at a single edge of the carrier 120 are used as reagent needles, thereby achieving a uniformly distributed structure for liquid suction from the liquid reservoir with optimized space utilization, it also facilitates a distribution of a plurality of fluid paths (such as in a form of a plurality of manifold branches) from the fluid guiding needles 123a to the selector valve 126, effectively avoiding mutual interference between different reagent paths and promoting regional thermal balance distribution.

In a further embodiment of the present disclosure, as shown in the figures, for example, a corresponding membrane breaking needle is provided aside each fluid guiding needle in one-to-one correspondence, and each fluid guiding needle is in pair with a corresponding single membrane breaking needle, and each fluid guiding needle and the corresponding single membrane breaking needle are adjacent to and spaced apart from each other.

In a specific embodiment, as an example, each fluid guiding needle includes a hollow and elongated straight tubular needle body, and a through terminal with a tapered longitudinal cross-section. For example, the plurality of fluid guiding needles are installed to the plurality of fluid guiding needle ports in a threaded connection manner.

In other embodiments of the present disclosure, as shown in the figures, for example, the fluid guiding component further includes: at least two guide pins protruding outward from a side of the top plate opposite to the chip receiving area and spaced apart from each other in a straight line, and configured to be in positive fit with alignment features on the reagent kit.

In a more specific embodiment, as shown in the figures, as an example, the at least two guide pins 123c are two guide pins 123c arranged collinear with the plurality of fluid guiding needles 123a, and located outside the two fluid guiding needles 123a at two ends of the plurality of fluid guiding needles 123a.

Through such arrangement, for example, the two guide pins 123c are aligned and in positive fit with corresponding alignment features on the liquid reservoir, such as pits, thereby serving as guides for accurately positioning all the fluid guiding needles 123a relative to the corresponding reagent dispensing ports in the liquid reservoir. In other words, once the two guide pins 123c at two ends are correctly aligned and fit into the alignment features on the liquid reservoir, all the fluid guiding needles 123a are positioned in alignment with respect to respective reagent dispensing ports.

In a specific embodiment, as an example, correspondingly, the plurality of fluid guiding needle ports are aligned in a straight line. In a more specific embodiment, as an example, the plurality of fluid guiding needle ports are spaced by a uniform spacing.

In addition, in embodiments of the present disclosure, for example, as shown in the figures, the selector valve is a rotary valve configured to be rotatable about an axis thereof to switch a selective communication between at least one of the plurality of inlet ports corresponding to the plurality of branch fluid passages and the outlet port via the fluid inlet 126a and the fluid outlet 126b.

In a further embodiment, preferably, an axial direction of the inlet port and an axial direction of the outlet port are parallel to the axis, the inlet port is offset from the axis, and the outlet port is coaxial with the axis.

In a specific embodiment, for example, the selector valve is installed to a back side of the top plate away from the chip receiving area via the valve seat 1261, and the plurality of inlet ports are arranged in a circular shape around the axis, the plurality of inlet ports and the outlet port are open to the back side of the top plate, and at least one of the plurality of inlet ports is selectively communicated to the fluid inlet 126a of the valve body 1262, and the outlet port is communicated to the fluid outlet 126b of the valve body 1262, and a center-to-center distance between each of the plurality of inlet ports and the outlet port is equal.

In a further embodiment, for example, the plurality of branch fluid passages diverge radially around the rotary valve to communicate with the plurality of fluid guiding needle ports in one-to-one correspondence, respectively.

Through such specific arrangements, the rotary valve 126 is used as the selector valve 126, and the plurality of inlet ports 1254 (preferably spaced apart from each other at a same angle in a circumferential direction) as outlets of the branch fluid passages 1252 are provided along a circumference direction of the rotary valve 126. Correspondingly, the plurality of branch fluid passages 1252 diverge radially outwardly from corresponding inlet ports 1254, for example, generally radially outward at uniform angular intervals around the axis AX of the rotary valve 126, thereby achieving a substantially uniformly spaced distribution of manifolds in a surrounding area adjacent to the rotary valve 126. This is convenient for a uniform distribution of the fluid path network 125 over an entire region between the rotary valve 126 and the fluid guiding needle ports 1250. An overall fluid path length is effectively controlled by planning the distribution of manifolds, redundant fluid path lengths are avoided, reagent loss is reduced, which is conducive to a temperature control and a temperature equalization throughout the fluid supply device 1.

Moreover, in embodiments of the present disclosure, as shown in the figures, the fluid path network 125 also achieves a centralized fluid supply through a single pipeline in the area between the selector valve 126 and the chip receiving area 121a (for accommodating the chip) of the carrier 120, simplifying the liquid supply arrangement in a final stage of supplying fluid, such as the reagent, to the chip.

In a more specific embodiment of the present disclosure, for example, the top plate includes: a top layer 120a, the chip receiving area is formed in the top layer 120a; a support layer 120b stacking with the top layer 120a and located between the top layer 120a and the selector valve. Moreover, the plurality of branch fluid passages and the common fluid passage are formed on a side of the support layer 120b towards the top layer 120a, and the plurality of inlet ports, the outlet port, and the plurality of fluid guiding needle ports extend to penetrate the support layer 120b.

More specifically, as shown in FIG. 6(a) to FIG. 6(c), for example, the chip receiving area of the top layer 120a is formed with a through accommodating hole 1202, and a recess 1203 recessing from a side of the top layer 120a away from the support layer 120b and arranged around the accommodating hole 1202.

As shown in FIG. 7, for example, the top plate further includes a chip adsorption component 120c partially fixed in the accommodating hole 1202 and protrude away from the support layer 120b from the accommodating hole 1202, and is configured to accommodate and adsorb the chip. The chip adsorption component includes: a pipe joint 1204 in communication with a negative pressure source; an adsorption table 1205 partially arranged in the accommodating hole 1202 with an edge of a top surface thereof protruding outward to define an adsorption slot 1206 in the top layer 120a recessing towards the support layer 120b and located between the edge and the top surface, the adsorption slot 1206 is constructed as a closed slot extending along the edge and in a closed loop form, and a shape and a size of the adsorption slot are determined to be suitable for surrounding and securing the edge of the chip; and a negative pressure channel 1207 penetratively formed in the adsorption table 1205 and communicated between the pipe joint 1204 and the adsorption slot 1206.

In embodiments of the present disclosure, for example, the top layer 14a is formed with a discharge port and a supply port penetrating therethrough and extending to the recess 1203. The supply port is in fluid communication with the selector valve via the common fluid passage, and the discharge port is spaced apart from the supply port.

Furthermore, for example, the common fluid passage is coupled between the outlet port and the supply port in a straight line.

For example, the plurality of branch fluid passages are arranged not to cross each other and to avoid the common fluid passage.

In a specific embodiment, for example, as shown in the figures, end portions of the discharge port and the supply port respectively extend to the recess 1203. As an example, as shown in FIG. 8, in a case that the chip is received and adsorbed on the adsorption table 1205, the adsorption table 1205 and the chip jointly define a chip liquid-tight sealing surface P1 as a first liquid-tight sealing surface, and the chip is in a sealed fluid communication with the supply port and the discharge port respectively at the first liquid-tight sealing surface.

In a specific embodiment, for example, as shown in the figures, the support layer 120b is formed with a discharge channel penetrating therethrough and in fluid communication from the discharge port to an outside of the top plate. As an example, as shown in FIG. 8, the plurality of inlet ports and the outlet port extend to penetrate the support layer 120b to the back side of the top plate, and the back side and the valve seat 1261 jointly define a valve liquid-tight sealing surface P2 as a second liquid-tight sealing surface, and the plurality of inlet ports and the outlet port respectively form a sealed fluid communication with the fluid inlet 126a and the fluid outlet 126b of the valve body 1262 at the second liquid-tight sealing surface.

The chip liquid-tight sealing surface P1 and the valve liquid-tight sealing surface P2 are respectively located on an upper side and a lower side of the fluid passages inside a switcher valve. Through such arrangement, a sealing surface configuration may be simplified, and by defining two sealing surfaces at different positions inside the carrier 120, easy fluid isolation and leak proof sealing at the chip and valve may be achieved.

Furthermore, as shown in the figures, for example, the chip platform 12 is provided with a driving component 122 inside, and the chip processing device 1 further includes, for example, at least one guide rail component 13 coupled between the accommodating chamber 110 and the substrate, and the accommodating chamber 110 is movable in a direction orthogonal to the substrate 10 via a transmission of the at least one guide rail component 13 by the driving component 122, so that the guide rail component 123 is inserted into and fluidly communicated to the liquid reservoir 2. As an example, as shown in FIG. 1(c), the chip processing device 1 includes two guide rail components 13 arranged in parallel.

In a specific embodiment of the present disclosure, the fluid guiding position is, for example, any position value within a range of a plurality of positions (referred to as a first position range). More specifically, once the accommodating chamber 110 is lifted to a first threshold position in the first position range (such as a lowest position in the first position range), the first fluid transport structure in the reagent kit platform 11 is engaged with the second fluid transport structure in the chip platform 12, thereby establishing the fluid communication between the first fluid transport structure and the second fluid transport structure.

Moreover, in a specific embodiment of the present disclosure, the non-fluid guiding position is also, for example, any position value within another range of a plurality of positions (referred to as a second position range). More specifically, once the accommodating chamber 110 is lowered to a second threshold position within the second position range (such as a highest position within the second position range), the first fluid transport structure in the reagent kit platform 11 is disengaged from the second fluid transport structure in the chip platform 12, thus the first fluid transport structure and the second fluid transport structure are separated from each other, and there is no longer fluid communication between the first fluid transport structure and the second fluid transport structure .

Typically, as an example, the first threshold position is higher than the second threshold position. Thus, the first position range and the second position range do not overlap with each other at all.

In a specific embodiment, for example, the driving component 122 includes: a driving source including one of a stepper motor and a piezoelectric driver; and a lead screw in transmission coupling between the driving source and at least one guide rail component 13. More specifically, as an example, the lead screw is further coupled to the at least one guide rail component 13 via a coupling located upstream of the lead screw and in transmission coupling with an output shaft of the driving source, thereby facilitating a reciprocating movement of the accommodating chamber 110 orthogonal to the substrate 10 via the transmission of the at least one guide rail component 13 by the driving component 122, i.e. achieving the lifting and the lowering action of the accommodating chamber 110.

In a specific embodiment, for example, each guide rail component 13 includes two spaced sets of cross roller guide rails 130, and each set of cross roller guide rails 130 includes a fixed rail 131 fixed to the chip platform 12, a movable rail 132 coupled to the accommodating chamber 110, and a plurality of rolling elements 135 held between the fixed rail 131 and the movable rail 132, such as a plurality of balls (in this case, a cross ball guide rail is served as each set of cross roller guide rails 130), or a plurality of pin rollers alternately arranged orthogonally (in this case, a cross pin roller guide rail is served as each set of cross roller guide rails 130), thereby facilitating carrying loads in a plurality of different directions. Moreover, in a further embodiment, as shown in the figures, in each set of cross roller guide rails 130, the fixed rail 131 is fixed to the chip platform 12 in a manner orthogonal to the substrate 10, and the movable rail 132 is fixed to the accommodating chamber 110 in a manner orthogonal to the substrate 10.

Thus, as shown in the figures, each set of cross rollers in at least one guide rail component 13 (illustrated as two guide rail components 13 arranged side-by-side) work together to serve as a guiding device for the accommodating chamber 110 to move in the direction orthogonal to the substrate 10, thereby achieving accurate guidance for the lifting and lowering of the accommodating chamber 110.

In a further embodiment, as an example, the lead screw is coupled with the respective movable rails 132 of the two sets of cross roller guide rails 130 in the at least one guide rail component 13. Through such arrangement between the respective movable rails 132 of different sets of cross roller guide rails 130 interconnected in each guide rail component 13, a synchronous guidance of the lifting action of the accommodating chamber 110 by the two sets of cross roller guide rails 130 spaced apart from each other in the same guide rail component 13 is achieved, and at the same time, it also facilitates maintaining an integrated support below the accommodating chamber 110 during a lifting period of the accommodating chamber 110.

As an example, the first fluid transport structure of the reagent kit platform 11 includes the liquid reservoir 2, such as the reagent kit.

As shown in FIG. 2(f), the chip platform 12 further includes a V-shaped support 133 for fixing the selector valve 126. The selector valve 126 is penetratively provided on the adapter bracket 127 which is fixed to a fixed bracket 12a. The V-shaped support 133 is connected between a bottom of the adapter bracket 127 and the selector valve 126, and the V-shaped support 133 includes two legs 1330 and a connecting portion 1332 that connects the two legs 1330. The two legs are connected to the bottom of the adapter bracket 127, for example, by a rotatable and adjustable threaded pair. The connecting portion 1332 may be fixed to the selector valve 126 through a similar threaded structure. Therefore, a stable installation of the selector valve in the chip platform 12 may be achieved.

FIG. 5(a) schematically shows a specific structure for transporting the fluid, such as the second fluid transport structure, in the top plate 120 of the chip platform 12 in the chip processing device 1 of FIG. 1. FIG. 5(b) schematically shows an assembly relationship between the top plate serving as the carrier and the selector valve.

As an example, the second fluid transport structure of the chip platform 12 is, for example, a fluid guiding device 160, which will be set forth below. More specifically, the fluid guiding device includes the fluid supply device 150 and the fluid guiding component 123. The fluid supply device includes the aforementioned top plate 120 (which serves as the carrier for carrying the sequencing chip), and a fluid path network 125 and a selector valve 126, whereby the top plate 120, the fluid path network 125, and the selector valve 126 together form the fluid supply device 150 for supplying the fluid to the chip. Furthermore, the fluid supply device 150 and the fluid guiding component 123 work together to jointly define the fluid guiding device 160 that serves as the second fluid transport structure.

Furthermore, the specific structure of the fluid transport device for transporting the fluid is disclosed in embodiments of the present disclosure. As an example, as shown in the figures, the chip processing device 1 further includes the fluid path network 125 formed in the top plate 120 of the chip platform 12, and the selector valve 126 installed to the top plate 120 and in fluid communication with the fluid path network. More specifically, the fluid path network 125 includes: a plurality of fluid guiding needle ports 1250 aligned in a straight line and spaced apart from each other; a plurality of branch fluid passages 1252, each branch channel 1252 having one end communicated to a corresponding fluid guiding needle port 1250 among the plurality of fluid guiding needle ports 1250, and the other end penetrating a side of the top plate 120 close to the selector valve 126 and selectively communicated with the fluid inlet 126a of the selector valve; and a single common fluid passage 1253 in fluid communication between the fluid outlet 126b of the selector valve 126 and the chip receiving area 121a, and the selector valve 126 is operable to switch an alignment and communication between a corresponding other end of one of the plurality of branch fluid passages 1252 and the fluid inlet 126a. The plurality of branch fluid passages 1252 collectively form a manifold section 1251.

The aforementioned top plate 120 serves as the carrier for carrying the sequencing chip, and in combination with the fluid path network 125 and the selector valve 126 as described above, the three together form the fluid supply device 150 that supplies the fluid to the chip. Accordingly, by using the specific structure of transporting the fluid provided in the top plate 120 of the chip platform 12 as described above, the fluid passages are mainly arranged in the top plate 120 of the chip platform 12, which facilitates shortening the length of the fluid passage, reducing a substitution ratio and an amount of the reagent used, and reducing a sequencing cost.

Moreover, in embodiments of the present disclosure, as shown in the figures, as an example, the selector valve 126 is a rotary valve configured to be rotatable about an axis AX thereof to cause the fluid inlet 126a to switch the alignment and fluid communication with one of the plurality of branch fluid passages 1252.

In a further embodiment, as shown in the figures, an axial direction of the fluid inlet 126a and an axial direction of the fluid outlet 126b are parallel to the axis AX, and the fluid inlet 126a is offset from the axis AX, and the fluid outlet 126b is coaxial with the axis AX.

In a more specific embodiment, as shown in the figures, as an example, the corresponding other ends of the plurality of branch fluid passages 1252 coupled with the fluid inlet 126a are configured as a plurality of inlet ports 1254 arranged in a circular shape around the axis AX, and a center-to-center distance between each inlet port 1254 and the fluid outlet 126b is equal to a center-to-center distance between the fluid inlet 126a and the fluid outlet 126b.

Thus, by using the rotary valve as the selector valve 126, it is convenient to selectively switch the fluid inlet 126a of the rotary valve to be in alignment with and in fluid communication with the outlet of a desired branch fluid passage 1252 in the plurality of branch fluid passages 1252, by simply rotating the valve body of the rotary valve.

FIG. 8 schematically shows a sectional view of the top plate serving as the carrier taken along the common fluid passage.

In another embodiment of the present disclosure, as shown in the figures, inside the rotary valve 126, the fluid inlet 126a and the fluid outlet 126b share the first sealing surface P1, and the plurality of inlet ports 1254 share the second sealing surface P2. The first sealing surface P1 and the second sealing surface P2 are respectively located on the upper and lower sides of the fluid passages inside the switcher valve.

Through such arrangement, the arrangement of the sealing surface is simplified, and easy fluid isolation is achieved by only having to define two non-overlapping sealing surfaces at different side fluid passages inside the valve body. This facilitates an effective liquid tight sealing and fluid passage blocking when the inlet port 1254 of the branch fluid passage 1252 is not aligned with the fluid inlet 126a of the rotary valve 126, and/or the inlet of the downstream common fluid passage 1253 is not aligned with the fluid outlet 126b of the rotary valve 126, ensuring timely opening and closing of the fluid passage at the rotary valve 126, as well as effective sealing when the rotary valve 126 is switched off so as to avoid unexpected mixing between the reagents.

FIG. 9(a) to FIG. 9(f) respectively show a fluid guiding device, particularly a fluid guiding component thereof, in the chip platform of the chip processing device of FIG. 1 from different angles. FIG. 9(a) and FIG. 9(b) are schematic perspective views of the fluid guiding device shown from different angles, respectively. FIG. 9(c) to FIG. 9(f) are a front view, a right view, a rear view, and a top view of the fluid guiding device, respectively.

In addition, in embodiments of the present disclosure, as shown in the figures, as an example, the plurality of fluid guiding needle ports 1250 are provided at a substantially longitudinal single edge 1208 of the top plate 120, and the plurality of branch fluid passages 1252 diverge radially around the selector valve 126 and turn towards the edge 1208 to communicate with the plurality of fluid guiding needle ports 1250 in one-to-one correspondence, respectively.

Through such a circumferential arrangement of ends of the branch fluid passages 1252 around the selector valve 126 (such as the rotary valve), typically e.g. at uniform angular spacing and circumferentially arranged to radially diverge in the radial direction, it is possible to achieve uniform arrangement of branch fluid passages 1252 in an adjacent peripheral area of the selector valve 126, thereby also facilitating an uniform temperature control of the entire area of the plurality of branch fluid passages 1252 distributed across the top plate 120 and an uniform spacing arrangement among the fluid passages.

Moreover, in embodiments of the present disclosure, as shown in the figures, the fluid guiding component 123 includes a plurality of fluid guiding needles 123a that are respectively in fluid communication with the plurality of branch fluid passages 1252 in one-to-one correspondence. The plurality of fluid guiding needles 123a protrude from a lower side of the aforementioned single edge 1208 of the top plate 120 of the chip platform 12 towards the reagent kit platform 11, and are spaced apart from each other (for example, spaced apart from each other at a uniform spacing) in a straight line. Each fluid guiding needle 123a is constructed as a hollow needle.

More specifically, for example, the plurality of fluid guiding needle ports 1250 are penetratively formed in a protruding portion 121b protruding from the edge 1208 of the top plate 120 towards a side of the top plate 120 away from the chip receiving area 121a accommodating the chip 3. Correspondingly, as an example, the plurality of fluid guiding needles 123a are installed in a threaded connection manner to the plurality of fluid guiding needle ports 1250 and are in fluid communication with the plurality of branch fluid passages 1252 in one-to-one correspondence. Therefore, the plurality of fluid guiding needle ports 1250 are also in the same arrangement as the plurality of fluid guiding needles 123a correspondingly. Specifically, for example, the plurality of fluid guiding needle ports spaced apart from each other (e.g. spaced apart from each other at the same uniform spacing as the fluid guiding needles) in a straight line at the single edge 1208 of the top plate 120.

The plurality of fluid guiding needles 123a are further configured, for example, to pierce a sealing pad covered above a corresponding reagent extraction site of the liquid reservoir 2 (e.g. the reagent kit) in the accommodating chamber 110 once the accommodating chamber 110 is lifted to an upper limit position in the reagent kit platform 11, thereby achieving fluid communication of the plurality of fluid guiding needles 123a to the corresponding reagent extraction sites in the liquid reservoir 2 in one-to-one correspondence. The upper limit position is, for example, the aforementioned fluid guiding position, for example, the highest position in the first position range that serves as the fluid guiding position.

A stable mechanical coupling and direct fluid communication between the fluid guiding needle 123a serving as the reagent needle (for suctioning the liquid from the reagent kit) and the fluid path network 125 are thereby achieved through a simple installation method.

In addition, in embodiments of the present disclosure, as shown in the figures, as an example, the common fluid passage 1253 is linearly coupled between the fluid outlet 126b and the chip receiving area 121a. And as an example, the plurality of branch fluid passages 1252 are arranged not to cross each other and avoid the common fluid passage 1253. Thus, for the selector valve 126, the fluid passage entering the selector valve 126 and the fluid passage output from the selector valve 126 are effectively separated from each other, and the selectively communicated branch fluid passages 1252 are also effectively spaced apart from each other.

In addition, in embodiments of the present disclosure, as shown in the figures, the fluid guiding component 123 further includes, for example, a plurality of membrane breaking needles 123b protruding outward from the lower side of the edge 1208 of the top plate 120 and are spaced apart from each other in a straight line. Each membrane breaking needle 123b is constructed as a solid needle. Furthermore, in a more specific embodiment, the plurality of fluid guiding needles 123a and the plurality of membrane breaking needles 123b are arranged parallel to each other, for example. Moreover, in a further embodiment, as an example, a corresponding membrane breaking needle 123b is arranged aside each of the plurality of fluid guiding needles 123a in one-to-one correspondence, and the membrane breaking needle 123b is configured to pierce the membrane for covering the sealing pad overlying the corresponding reagent extraction site of the liquid reservoir 2 (e.g. the reagent kit) in the accommodating chamber 110 once the accommodating chamber 110 is lifted to the upper limit position in the reagent kit platform 11. And more specifically, for example, each fluid guiding needle 123a is in pair with a corresponding single membrane breaking needle123b, and each fluid guiding needle and the corresponding single membrane breaking needle are adjacent to and spaced apart from each other.

In addition, in another embodiment of the present disclosure, the fluid guiding component 123 further includes, for example, at least two guide pins 123c protruding outward from the lower side of the edge 1208 of the top plate 120 and are spaced apart from each other in a straight line, and are configured to be in positive fit with the alignment feature on the liquid reservoir 2. After the guide pin 123c and the alignment feature are aligned with each other, the guide pin 123c is inserted into the alignment feature (such as an alignment hole or an alignment recess) to achieve the assembly and positioning of the liquid reservoir 2 and the fluid guiding component 123. In a more specific embodiment, for example, the at least two guide pins 123c are two guide pins 123c arranged collinear with the plurality of fluid guiding needles 123a and located outside two fluid guiding needles 123a at two ends of the plurality of fluid guiding needles 123a.

In another embodiment of the present disclosure, as an example, the fluid path network 125 further includes an outlet pipeline for discharging a waste liquid and a return pipeline for returning an injected reagent, which are respectively communicated to the chip receiving area 121a.

By utilizing the specific structure of the chip processing device 1 for transporting the fluid as described above, a ductless fluid path from the reagent kit serving as the liquid reservoir 2 to the sequencing chip 3 may be achieved. Moreover, according to the specific structure of embodiments of the present disclosure, liquid suction from the top of the reagent kit may be achieved, thereby avoiding long stroke translating and lifting movements of the reagent kit, as well as avoiding the insertion of the reagent needle into the bottom of the reagent kit. Meanwhile, by combining with other structures of the chip processing device 1, precise positioning of the reagent kit may be achieved.

Thus, the fluid guiding component 123 and the aforementioned fluid supply device 150 (including the aforementioned top plate 120 serving as the carrier for carrying the sequencing chip, the fluid path network 125, and the selector valve 126) jointly define the fluid guiding device 160, which is configured to guide various reagent fluids from the fluid reservoir (such as the reagent kit 2) to the sequencing chip 3.

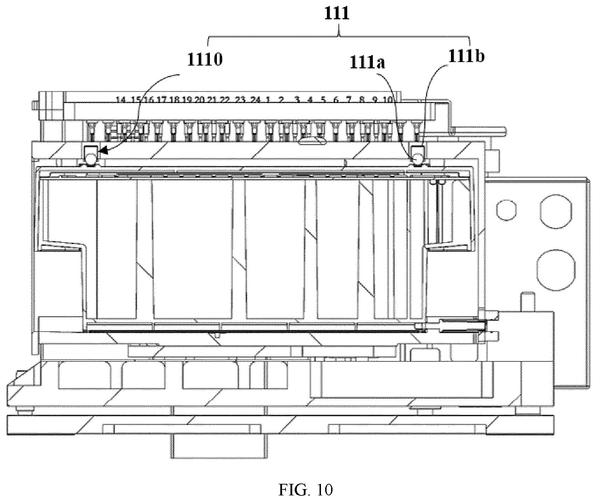

In addition, FIG. 10 shows a sectional view of the chip processing device 1 shown in the top view of FIG. 2(e) taken along line E-E, in which an internal positioning device 111 and an in-position detector 112 used to detect in-position of the insertion of the liquid reservoir 2 inside the accommodating chamber 110 are illustrated.

As shown in the figures, in embodiments of the present disclosure, for example, the chip processing device 1 further includes a positioning device 111, and the positioning device 111 is, for example, arranged in the reagent kit platform 11, and includes: a groove 1110 concavely formed in a top-side inner wall of the accommodating chamber 110; an elastic component arranged in the groove 1110; and a positioning bead 111b arranged at an end of the elastic component towards the substrate and configured to: in response to a situation where the reagent kit does not reach the positioning bead inside the accommodating chamber, the elastic component is in an initial state not subjected to a force applied by the reagent kit, and the positioning bead protrudes at least partially towards the substrate from the top-side inner wall of the accommodating chamber; and in response to a situation where the liquid reservoir 2 is inserted into the accommodating chamber 110 and the positioning bead is pressed, the elastic component is pushed towards the groove via the positioning bead, thereby causing the elastic component to retract at least partially back into the groove 1110. With this arrangement of the positioning device 111, when the liquid reservoir 2 such as the reagent kit is inserted into the accommodating chamber 110, the liquid reservoir 2 gradually pushes the positioning bead 111b of the elastic component exposed from the groove 1110 toward an interior of the groove 1110, until the liquid reservoir 2 is inserted in position inside the accommodating chamber 110, and the positioning bead 111b is pressed outward against a surface of the liquid reservoir 2 by an elastic restoring force of a spring 111a in a compressed state, thereby achieving a firm positioning of the liquid reservoir 2 so as to keep the liquid reservoir 2 in position inside the accommodating chamber 110.

In a more specific embodiment, for example, a top wall 25 of the reagent kit has a protruding portion 23 protruding towards the top-side inner wall of the accommodating chamber, and a positioning groove 24 recessing from the protruding portion 23, and in response to a situation where the reagent kit is inserted into the accommodating chamber and the positioning bead is pressed, the positioning bead is clamped between the positioning groove 24 and the elastic component. In other words, the surface of the liquid reservoir 2 is further formed with at least one recess provided corresponding to a position of the positioning bead 111b when the liquid reservoir 2 is in position inside the accommodating chamber 110, and a shape and a size of the recess are determined to be suitable for receiving the positioning bead 111b. Therefore, when the liquid reservoir 2 is inserted in position, the positioning bead 111b is pushed outward by the elastic restoring force of the compressed spring 111a to cooperate with the recess, thereby locking the liquid reservoir 2 in position in an elastic manner. This elastic locking will not affect the removal of the reagent kit from the accommodating chamber 110 after subsequent sequencing work is completed.

In embodiments according to the present disclosure, as an example, the chip processing device 1 further includes an in-position detector 112 inside the accommodating chamber 110, the in-position detector 112 includes an optocoupler component and a shielding member, the shielding member is, for example, arranged on or coupled with the liquid reservoir 2, the optocoupler component includes an infrared emitter and an infrared receiver. The optocoupler components are arranged inside the reagent kit platform 11 and spaced apart from each other, and are configured to determine that the liquid reservoir 2 is in position inside the accommodating chamber 110 in response to the shielding member being displaced or deformed by a force applied by the liquid reservoir 2 and reception of infrared rays from the infrared emitter by the infrared receiver being blocked when the liquid reservoir 2 is inserted in position inside the accommodating chamber 110. By using the shielding member carried by the liquid reservoir 2, event detection and determination of whether the liquid reservoir 2 is in position in the accommodating chamber 110 is facilitated.

In an alternative embodiment of the present disclosure, as an example, the chip processing device 1 further includes a positioning feature provided on an outer surface of the liquid reservoir 2, and an in-position sensor provided inside the accommodating chamber 110. The positioning feature is aligned with the in-position sensor when the liquid reservoir 2 is inserted in position inside the accommodating chamber 110, and the in-position sensor is configured to determine that the liquid reservoir 2 has been in position inside the accommodating chamber 110 by detecting a pushing action applied by the positioning feature. Furthermore, for example, the in-position sensor is a force sensor. Bu using such alternative arrangement, the in-position detection of the liquid reservoir 2 inserted into the accommodating chamber 110 is achieved based on contact force using the force sensor.

In embodiments of the present disclosure, referring back to FIG. 1, as an example, the chip processing device 1 further includes a pressing device 113 formed at the top of the reagent kit platform 11. The top of the reagent kit platform 11 is formed with a through opening to at least partially expose the liquid reservoir 2 inserted into the accommodating chamber 110. The pressing device 113 includes a platen 1130, one side of the platen 1130 is relatively fixed to the substrate 10; a pin 1131 penetrating a free side of the platen 1130 opposite to the one side; a pair of arms 1132, each arm 1132 having one end rotatably coupled to a corresponding end of the two ends of the pin 1131; and a pair of elastic members 1133, each elastic member 1133 (such as a torsion spring) is elastically coupled between a corresponding arm 1132 of the pair of arms 1132 and the reagent kit platform 11. With this arrangement of the pressing device 113, once the liquid reservoir 2 is inserted in position inside the accommodating chamber 110, for example, by slightly pushing one of the pair of arms 1132 around the pin 1131, further pivoting of the pair of arms 1132 around the pin 1131 under the elastic force of the coupled pair of springs may be triggered, achieving pressing against top of the accommodating chamber 110 and the liquid reservoir 2 using the platen 1130, thereby further pressing the accommodating chamber 110 and the liquid reservoir 2 downward in a substantially vertical direction.

In embodiments of the present disclosure, referring back to FIG. 1, further, for example, additional tilt adjustment structures are provided to facilitate leveling of the reagent kit platform 11 and the chip platform 12. More specifically, as shown in FIG. 1(d) to FIG. 1(f), as an example, the chip platform 12 includes a fixed bracket 12a, and the fixed bracket 12a is supported and fixed between the top plate 120 and the substrate 10, and partially extends between the reagent kit platform and the substrate 10 and leaves a gap with the reagent kit platform 11. An extension plate 12b fixed to the platen 1130 extends from a side edge of the fixed bracket 12a corresponding to the reagent kit platform 11, and the reagent kit platform 11 is located between the extension plate 12b and the selector valve. The reagent kit platform 11 includes a movable bracket 11a, and the reagent chamber 110 is suspended and supported by the movable bracket 11a. The movable bracket 11a is fixed to the movable rail 132 and movably connected to the fixed rail 135 through the movable rail 132. Preferably, the movable bracket 11a is L-shaped.

The chip processing device 1 further includes a first tilt adjustment mechanism 114 arranged between the fixed bracket 12a and the substrate 10 and located on a side of the reagent kit platform 11. The first tilt adjustment mechanism 114 includes a first fix distance head and two first threaded pairs 1140 being rotatable and adjustable relative to the substrate 10 that are arranged in a non-straight line. In addition, as an example, the chip processing device 1 further includes a second tilt adjustment mechanism 124 arranged between the fixed bracket 12a and the substrate 10 and located on a side of the chip platform 12. The second tilt adjustment mechanism 124 includes a second fix distance head and two second threaded pairs 1240 being rotatable and adjustable relative to the substrate 10 that are arranged in a non-straight line. Due to the fact that the first fix distance head and the two adjustable first threaded pairs 1140 in the first tilt adjustment structure jointly define a three-point support structure, a stable support is achieved once the first threaded pair 1140 is adjusted to level the reagent kit platform 11. Similarly, due to the fact that the second fix distance head and the two adjustable second threaded pairs 1240 in the second tilt adjustment structure jointly define another three-point support structure, a stable support is achieved once the second threaded pair 1240 is adjusted to level the chip platform 12.

In embodiments of the present disclosure, referring back to FIG. 2(b) and FIG. 2(d), for example, the chip processing device 1 further includes a position limiting device 14, including an induction sheet 140 arranged on a corresponding movable rail 132 of at least one set of cross roller guide rails 130 in at least one guide rail component 13; and an upper position limiting optocoupler 141 and a lower position limiting optocoupler 142 respectively arranged at positions corresponding to upper and lower ends of a stroke of the corresponding movable rail 132 at two ends of the corresponding fixed rail 131 of the at least one set of cross roller guide rails 130, and are respectively configured to stop the driving source in response to determining that the movable rail 132 is lifted to an upper limit position by detecting that the upper position limiting optocoupler 141 is blocked by the induction sheet 140, and to stop the driving source in response to determining that the movable rail is lowered to a lower limit position by detecting that the lower position limiting optocoupler 142 is blocked by the induction sheet 140. The lower limit position is, for example, the aforementioned non-fluid guiding position, for example, the lowest position in the second position range that serves as the non-fluid guiding position.

Through the above-described specific arrangement of the position limiting device 14, the upper and lower limit positions of the accommodating chamber 110 during the lifting movements of the liquid reservoir 2 carried and inserted inside the accommodating chamber 110 on the reagent kit platform 11 may be detected and determined, and the power supply of the driving source may be cut off once the accommodating chamber 110 reaches the upper or lower limit position, thereby achieving limit position detection and position limiting in an electronically controlled manner.

Additionally, as an extended embodiment of the present disclosure, the position limiting device further includes two position limiting blocks respectively arranged at two ends of the fixed rail 131 of at least one set of cross roller guide rails, and each position limiting block is at least partially aligned with the corresponding movable rail 132. Thus, once the accommodating chamber 110 is lifted to the upper limit position or lowered to the lower limit position, the position limiting block may realize an auxiliary position limiting in a mechanical manner.

In the chip processing device 1, in coordination with the specific structure for transporting the fluid described above, there is further provided a power component 129, and the power component 129 is arranged in fluid communication with the fluid path network and configured to drive the fluid to flow through the fluid path network towards the supply port.

As an example, the power component includes a pump in communication with the chip receiving area, and the pump is configured to provide a negative pressure to the chip receiving area. For example, the pump is an injection pump at least arranged on at least one of upstream and downstream of the selector valve 126, and the injection pump is configured to provide a negative pressure to the chip receiving area 121a. Thus, the suction of the fluid is performed by the negative pressure provided by the injection pump, and the suction of the fluid (more specifically, for example, the reagent in the reagent kit) may be achieved without inserting the fluid guiding needle 123a into the bottom of the liquid reservoir 2, thereby providing fluid suction and liquid supply from the reagent kit to the sequencing chip 3.

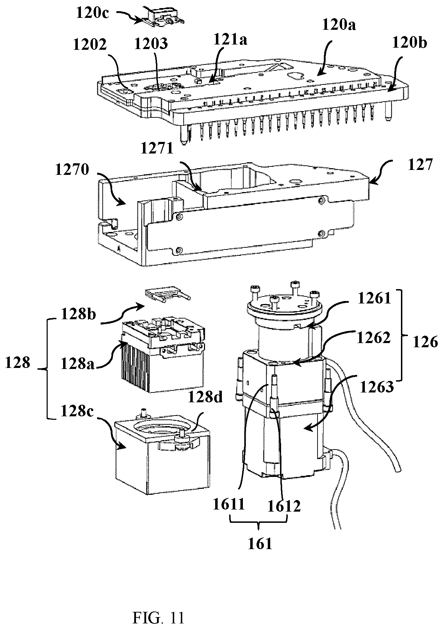

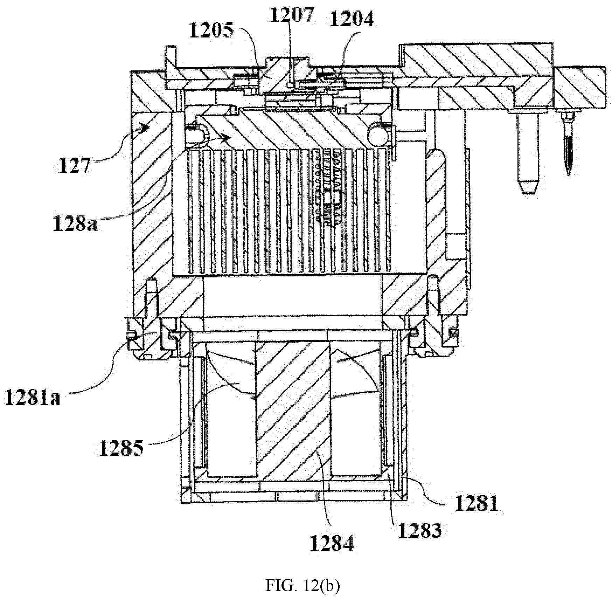

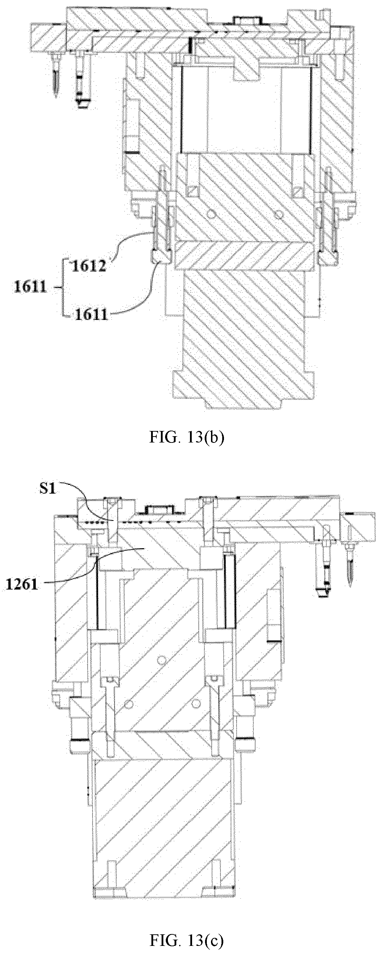

FIG. 11 shows an assembly relationship of a temperature control component relative to the carrier via the adapter bracket in an exploded view. FIG. 12(a) shows a sectional view taken along line A-A in FIG. 9(f), and FIG. 12(b) shows a sectional view taken along line B-B in FIG. 9(f), particularly showing a configuration and an assembly relationship of the temperature control component, the temperature control component being assembled with the top plate through screw. FIG. 13(b) to FIG. 13(c) respectively show sectional views taken along line C-C and line D-D in FIG. 9(f), particularly showing an assembly relationship between the selector valve and the top plate connected through a screw S1.

As an example, the fluid guiding device 160 is, for example, additionally integrated with a temperature control component 128, and the temperature control component is arranged on a side of the top plate opposite to the chip receiving area and configured to regulate a temperature of the fluid supplied from the fluid guiding component to the chip receiving area.

In addition, in embodiments of the present disclosure, as an example, the temperature control component 128 is arranged below the top plate 120 (more specifically, on the opposite side surface of the chip receiving area 121a for receiving the chip 3), and is configured to regulate the temperature of the fluid supplied from the fluid guiding component 123 to the chip receiving area 121a.

In a specific embodiment of the present disclosure, for example, the temperature control component 128 includes a first temperature adjustment device 128a adjacent to the selector valve on the same side of the top plate. As an example, as shown in the figures, the fluid guiding device further includes an adapter bracket 127 fixed to a side of the top plate opposite to the chip receiving area and constructed in a frame form. The adapter bracket 127 is formed with two concave cavities 1270, 1271 arranged side-by-side and recessing from opposite sides of the adapter bracket 127 away from the top plate and towards the top plate, respectively, for accommodating and fixing the selector valve and the first temperature adjustment device therein, respectively.

Through such arrangement, it is further ensured that the selector valve is reliably fixed relative to the carrier, and the assembly relationship of the first temperature adjustment device being fixed relative to the carrier 120 via the adapter bracket 127 is also achieved.

As an example, as shown in the figures, the first temperature adjustment device 128a includes: a heat dissipation component accommodated and installed in one of the two concave cavities 1270, 1271 of the adapter bracket 127 recessing from a side towards the top plate, including at least one of an active heat dissipation component and a passive heat dissipation component; and a temperature control module 1282 installed to the heat dissipation component and configured to cut off the power component 129 when a temperature at the heat dissipation component exceeds a threshold temperature.

As an example, the temperature control component further includes a heat conducting member 128b (also illustrated as being located inside the adapter bracket 127) inserted between the carrier and the first temperature adjustment device. More specifically, for example, the heat conducting member 128b includes a phase change material.

In a more specific embodiment, for example, the active heat dissipation component includes a thermoelectric cooler (e.g., a thermoelectric cooler (TEC) such as a Peltier effect device, a fan, and the like); or the passive heat dissipation component includes a heat sink, such as a fin heat sink, formed as a single heat sink or a heat sink array.

In an additional embodiment of the present disclosure, as an example, the temperature control component 128 further includes a second temperature adjustment device 128c, and the second temperature adjustment device is fixed to a side of the adapter bracket 127 away from the top plate and arranged side-by-side with the rotary valve, and is aligned with the first temperature adjustment device.

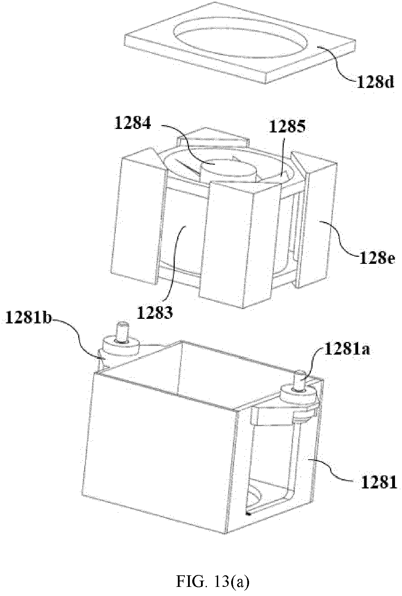

In combination with FIG. 13(a), in embodiments of the present disclosure, the second temperature adjustment device includes a fan 1280. The fan 1280 includes a hollow first housing 1281 defining a cavity inside for air flow, the first housing 1281 is arranged such that the cavity is aligned with the first temperature adjustment device and open towards the first temperature adjustment device to fluidly communicate the cavity between the first temperature adjustment device and an outside of the fluid guiding device; and a fan component accommodated inside the first housing 1281.

In a further embodiment, for example, the fan component includes: a second housing 1283 constructed as a hollow cylindrical body fixedly sleeved inside the first housing 1281; a rotating shaft 1284 rotatably installed inside the second housing 1283; a plurality of fan blades 1285 coaxially fixed to the rotating shaft 1284 inside the second housing 1283 and rotatable with the rotating shaft 1284 relative to the second housing 1283.

In embodiments of the present disclosure, the adapter bracket 127 is further formed with a gas channel 1286 penetrating an inside thereof and open at opposite ends respectively towards cavities of the first temperature adjustment device and the second temperature adjustment device, and the cavities are communicated to the first temperature adjustment device via the gas channel 1286.

Therefore, the fan is located on a side of the heat dissipation component of the first temperature adjustment device away from the carrier 120, which facilitates accelerating an airflow exchange and thus improving heat exchange speed and efficiency of the heat dissipation component.

In embodiments of the present disclosure, for example, the temperature control module 1282 is a temperature control switch, and the temperature control switch 1282 is configured to cut off the power supply from the power component 129 when the temperature at the heat dissipation component exceeds a threshold temperature.

Through the above-mentioned arrangement, it is convenient to achieve effective temperature control for the chip receiving area 121a, especially around the selector valve 126.

In embodiments of the present disclosure, as an example, the second temperature adjustment device 128c further includes a vibration reduction device, and the vibration reduction device includes a gasket located between the adapter bracket 127 and the first housing 1281 of the fan 1280; and a second level vibration reduction structure arranged inside the first housing 1281 of the fan 1280 between the first housing 1281 and the second housing 1283 of the fan component, the second level vibration reduction structure includes a plurality of vibration reduction members respectively snap-fitted to an inner wall of the first housing 1281 and spaced apart from each other, and the second housing 1283 of the fan component is coupled to the first housing 1281 via the plurality of vibration reduction members.

More specifically, as an example, the first housing of the fan is fixed to the adapter bracket via a threaded member 1281a of a flexible vibration absorbing material, and the first housing 1281 is convexly formed with a lug 1281b for installing the threaded member 1281a. A first level vibration reduction structure is pressed between the adapter bracket 127 and the first housing 1281 via the threaded member 1281a.

In a further embodiment, for example, at least one of the first level vibration reduction structure 128d and the second level vibration reduction structure 128e is a vibration compensation device, and the vibration compensation device is an elastic member or a damping member.

In a more specific embodiment, the first housing 1281 of the fan 1280 is fixed to the adapter bracket 127, for example, via a threaded member of a flexible vibration absorbing material, and the first level vibration reduction structure is pressed between the adapter bracket 127 and the first housing 1281 via the threaded member.

Therefore, through the above-mentioned arrangement, for the second temperature adjustment device 128c including, for example, a fan for providing forced air cooling, a vibration reduction structure arranged in a sandwich pattern (such as the illustrated two-level vibration reduction structure arranged separately from each other) is further adopted. By inserting the second temperature adjustment device 128c between the first level vibration reduction structure 128d and the second level vibration reduction structure 128e, a sandwich style multi-level vibration reduction structure is achieved, which may effectively counteract, or at least partially dissipate and block the vibration caused by the fan 128c for providing forced air cooling, and avoid vibration transmission from the temperature control component 128, especially the aforementioned second temperature adjustment device 128c including the fan 128c, to other parts such as the fluid guiding device 160 (such as, but not limited to, the branch fluid passage 1252 and the common fluid passage 1253 of the fluid path network 125, and the selector valve 126).

In addition, in embodiments of the present disclosure, as shown in FIG. 13(b) to FIG. 13(c), for example, in embodiments of the present disclosure, as shown in the figures, for example, the selector valve 126 is fixed to the carrier via a threaded connection at the valve seat 1261 of the selector valve 126, and the selector valve is coupled to the adapter bracket 127 via an adjustable abutting device 161, and the adjustable abutting device 161 includes a plurality of fix distance screws 1611 respectively threadably penetrating the adapter bracket 127 and abutting against the valve body 1262 of the selector valve; and a plurality of springs 1612 elastically abutting against the plurality of fix distance screws towards the top plate in one-to-one correspondence, respectively.

Therefore, the selector valve 126 is supported to the carrier 120, for example in a suspended and floating like manner, and the spring 1612 forms a structure similar to an elastic suspension, which works in combination with the fix distance screw 1611 to jointly carry the selector valve 126. In addition, the spring 1612 inside the adjustable abutting device 161 also simultaneously assists in filtering vibrations from a vibration source (such as the fan 128c and the active heat dissipation component) coupled directly or indirectly.

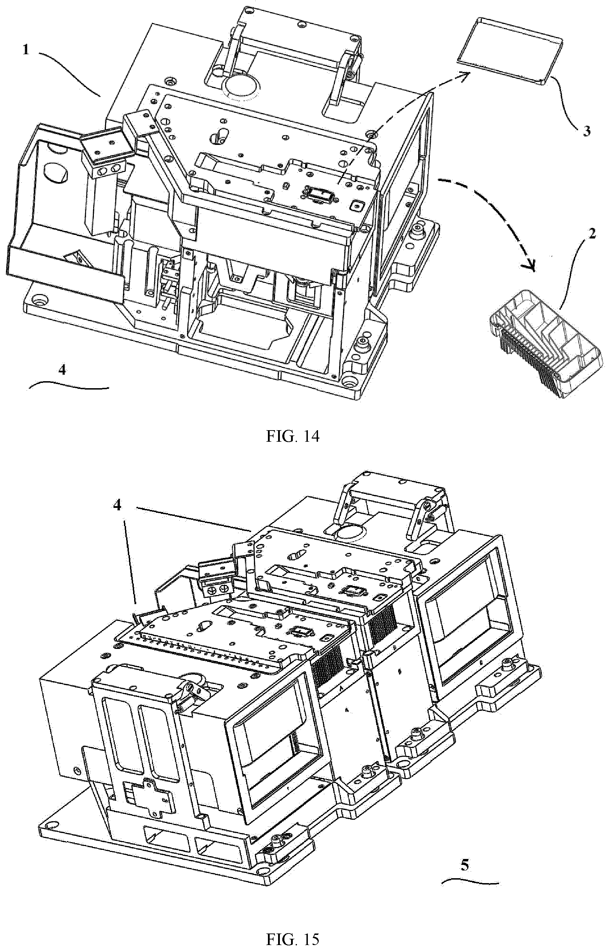

FIG. 14 schematically shows a gene sequencer 4 according to an embodiment of the present disclosure, including: a chip carrying a sample for fluid detection, and the aforementioned chip processing device.

According to another aspect of embodiments of the present disclosure, based on the overall technical concept of embodiments of the present disclosure, for example, as shown in FIG. 14, there is further provided a gene sequencer 4. The chip processing device includes a substrate extending in a first direction, and a reagent kit platform and a chip platform assembled side-by-side and adjacently on the substrate in a second direction transverse to the first direction. a top plate on a side of the chip platform away from the substrate has a chip receiving area for accommodating a chip, the reagent kit platform is formed with a hollow accommodating chamber, and the reagent kit is received in the accommodating chamber and at least partially filled with a fluid inside. For example, the reagent kit has a first fluid transport structure located on a side of the reagent kit towards the chip platform in the second direction, and the chip platform has a second fluid transport structure located on a side of the chip platform towards the reagent kit platform in the second direction and configured to at least partially overlap and communicate with the first fluid transport structure in a case that the reagent kit platform is assembled with the chip platform. The reagent kit is removably inserted into the accommodating chamber, and the fluid in the reagent kit is in fluid communication with the sample carried on the chip via the first fluid transport structure and the second fluid transport structure.

In a more specific embodiment, the accommodating chamber is coupled to the substrate in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively. The chip processing device is configured to: in response to the accommodating chamber being lifted away from the substrate to the fluid guiding position, the first fluid transport structure is engaged with the second fluid transport structure for fluid communication; and in response to the accommodating chamber being lowered towards the substrate to the non-fluid guiding position, the first fluid transport structure is separated from the second fluid transport structure.

As an example, more specifically, the top plate on a side of the chip platform away from the substrate has a flange protruding towards the reagent kit platform in the second direction, and the reagent kit platform is partially embedded and assembled between the flange of the top plate and the substrate.

As an example, more specifically, the first fluid transport structure includes a plurality of reagent slots 20a and a plurality of guide slots 20b arranged in one-to-one correspondence inside the reagent kit, and a communication channel 20c in fluid communication between a bottom of each guide slot 20b and a bottom of a corresponding reagent slot 20a. Each reagent slot 20a is at least partially filled with the fluid and has a first opening 21 open upward towards the flange and a first pierceable structure 210 covering the first opening 21, and each guide slot 20b has a second opening 22 open upward towards the flange, and a second pierceable structure 220 covering the second opening 22.

As an example, more specifically, the second fluid transport structure includes a fluid supply device, including a fluid path network formed in the top plate of the chip platform and communicated between the first fluid transport structure and the chip, and a selector valve installed to the top plate and in fluid communication with the fluid path network.

As an example, more specifically, the selector valve includes a valve seat 1261, the selector valve is fixed to the top plate via the valve seat 1261; a valve body 1262 extending from the valve seat 1261 in a direction away from the top plate and formed with a fluid inlet 126a configured to guide the fluid to flow into an interior of the valve body 1262 and a fluid outlet 126b configured to guide the fluid to flow outward from the interior of the valve body 1262. As an example, the fluid path network includes: a plurality of fluid guiding needle ports located on a side of the top plate opposite to the chip receiving area; a plurality of inlet ports spaced apart from the plurality of fluid guiding needle ports in one-to-one correspondence; a plurality of branch fluid passages each being communicated between each fluid guiding needle port and a corresponding inlet port, and being configured to guide the fluid input from each fluid guiding needle port to the corresponding inlet port; an outlet port spaced apart from the plurality of inlet ports and not in communication with the plurality of branch fluid passages; and a common fluid passage communicated between the outlet port and the chip receiving area, and configured to guide the fluid output from the outlet port to the chip receiving area. For example, the selector valve is arranged such that the fluid outlet 126b is in fluid communication with the outlet port, and the fluid inlet 126a is in fluid communication with at least one of the plurality of inlet ports, and the selector valve is configured to switch a selective communication between at least one of the plurality of inlet ports corresponding to the plurality of branch fluid passages and the outlet port via the fluid inlet 126a and the fluid outlet 126b.

As an example, more specifically, the plurality of fluid guiding needle ports are penetratively formed in a protruding portion at an edge of the top plate protruding towards a side of the top plate away from the chip receiving area.

As an example, more specifically, the second fluid transport structure further includes a fluid guiding component protruding from the flange towards the reagent kit platform and being communicated to the chip receiving area, and the fluid guiding component includes: a plurality of membrane breaking needles and a plurality of fluid guiding needles, respectively protruding from a side of the top plate opposite to the chip receiving area towards the reagent kit platform, each membrane breaking needle has a first end aligned with a corresponding first pierceable structure 210, and each fluid guiding needle has a second end aligned with a corresponding second pierceable structure 220. For example, each membrane breaking needle is not connected to the fluid path network and is configured to, in response to the accommodating chamber reaching the fluid guiding position, pierce the first pierceable structure 210 with the first end thereof and then insert into the reagent slot 20a to expose the reagent slot 20a so as to change an air pressure inside the reagent slot 20a. Each fluid guiding needle is constructed as a hollow needle, in fluid communication with a corresponding branch fluid passage of the plurality of branch fluid passages in one-to-one correspondence, and is configured to, in response to a situation where the accommodating chamber reaches the fluid guiding position, pierce the second pierceable structure 220 with the second end thereof and then insert into the guide slot 20b for fluid communication to the guide slot 20b so as to draw the fluid in the guide slot 20b.

As an example, more specifically, in a case that the plurality of fluid guiding needles are inserted into the guide slot 20b, a free end of each of the plurality of fluid guiding needles is higher than an inner wall at a bottom of the reagent slot 20a.

As an example, more specifically, each guide slot 20b is constructed as a slot having a circular cross-section with a first inner diameter, and each guide slot 20b is constructed as a slot having a circular cross-section with a second inner diameter smaller than the first inner diameter.

As an example, more specifically, the first pierceable structure is a metal foil; and the second pierceable structure is a flexible sealing element, such that a liquid-tight seal is maintained after being pierced by a corresponding fluid guiding needle of the plurality of fluid guiding needles.

Since the gene sequencer 4 includes the aforementioned chip processing device 1, the gene sequencer 4 also possesses all the advantages of the chip processing device 1, which will not be described in details herein.

FIG. 15 shows a gene sequencing apparatus 5 according to an embodiment of the present disclosure, including a pair of aforementioned gene sequencers 4 arranged in mirror symmetry and adjacent to each other.

According to another aspect of embodiments of the present disclosure, based on the overall technical concept of embodiments of the present disclosure, for example, as shown in FIG. 15, there is further provided a gene sequencing apparatus 5, including: a chip carrying a sample for fluid detection; and at least two aforementioned chip processing devices, each including a substrate extending in a first direction, and a reagent kit platform and a chip platform assembled side-by-side and adjacently on the substrate in a second direction transverse to the first direction, a top plate of the chip platform on a side away from the substrate has a chip receiving area for accommodating the chip, the reagent kit platform is formed with a hollow accommodating chamber, and the reagent kit is received in the accommodating chamber and at least partially filled with the fluid inside. For example, the reagent kit has a first fluid transport structure located on a side of the reagent kit towards the chip platform in the second direction, and the chip platform has a second fluid transport structure located on a side of the chip platform towards the reagent kit platform in the second direction and configured to at least partially overlap and communicate with the first fluid transport structure in a case that the reagent kit platform is assembled with the chip platform. For example, the reagent kit is removably inserted into the accommodating chamber, and the fluid in the reagent kit is in fluid communication with the sample carried on the chip via the first fluid transport structure and the second fluid transport structure. Specifically, as shown in the figures, the at least two chip processing devices are arranged symmetrically with respect to each other and the respective chip platforms are arranged adjacent to each other.

As an example, more specifically, the at least two chip processing devices include at least one pair of chip processing devices arranged in mirror symmetry with the respective chip platforms arranged adjacent to each other.

As an example, more specifically, the accommodating chamber is coupled to the substrate in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively. For example, the chip processing device is configured to: in response to the accommodating chamber being lifted away from the substrate to the fluid guiding position, the first fluid transport structure is engaged with the second fluid transport structure for fluid communication; and in response to the accommodating chamber being lowered towards the substrate to the non-fluid guiding position, the first fluid transport structure is separated from the second fluid transport structure.

As an example, more specifically, the top plate on a side of the chip platform away from the substrate has a flange protruding towards the reagent kit platform in the second direction, and the reagent kit platform is partially embedded and assembled between the flange of the top plate and the substrate.

As an example, more specifically, the first fluid transport structure includes a plurality of reagent slots 20a and a plurality of guide slots 20b arranged in one-to-one correspondence inside the reagent kit, and a communication channel 20c in fluid communication between a bottom of each guide slot 20b and a bottom of a corresponding reagent slot 20a. Each reagent slot 20a is at least partially filled with the fluid and has a first opening 21 open upward towards the flange and a first pierceable structure 210 covering the first opening 21, and each guide slot 20b has a second opening 22 open upward towards the flange and a second pierceable structure 220 covering the second opening 22.

As an example, more specifically, the second fluid transport structure includes a fluid supply device, including a fluid path network formed in the top plate of the chip platform and communicated between the first fluid transport structure and the chip, and a selector valve installed to the top plate and in fluid communication with the fluid path network.

As an example, more specifically, the selector valve includes a valve seat 1261, the selector valve is fixed to the top plate via the valve seat 1261; a valve body 1262 extending from the valve seat 1261 in a direction away from the top plate and formed with a fluid inlet 126a configured to guide the fluid to flow into an interior of the valve body 1262 and a fluid outlet 126b configured to guide the fluid to flow outward from the interior of the valve body 1262. As an example, the fluid path network includes: a plurality of fluid guiding needle ports located on a side of the top plate opposite to the chip receiving area; a plurality of inlet ports spaced apart from the plurality of fluid guiding needle ports in one-to-one correspondence; a plurality of branch fluid passages each being communicated between each fluid guiding needle port and a corresponding inlet port, and being configured to guide the fluid input from each fluid guiding needle port to the corresponding inlet port; an outlet port spaced apart from the plurality of inlet ports and not in communication with the plurality of branch fluid passages; and a common fluid passage communicated between the outlet port and the chip receiving area, and configured to guide the fluid output from the outlet port to the chip receiving area. For example, the selector valve is arranged such that the fluid outlet 126b is in fluid communication with the outlet port, and the fluid inlet 126a is in fluid communication with at least one of the plurality of inlet ports, and the selector valve is configured to switch a selective communication between at least one of the plurality of inlet ports corresponding to the plurality of branch fluid passages and the outlet port via the fluid inlet 126a and the fluid outlet 126b.

As an example, more specifically, the plurality of fluid guiding needle ports are penetratively formed in a protruding portion at an edge of the top plate protruding towards a side of the top plate away from the chip receiving area.

As an example, more specifically, the second fluid transport structure further includes a fluid guiding component protruding from the flange towards the reagent kit platform and being communicated to the chip receiving area, and the fluid guiding component includes: a plurality of membrane breaking needles and a plurality of fluid guiding needles, respectively protruding from a side of the top plate opposite to the chip receiving area towards the reagent kit platform, each membrane breaking needle has a first end aligned with a corresponding first pierceable structure 210, and each fluid guiding needle has a second end aligned with a corresponding second pierceable structure 220. For example, each membrane breaking needle is not connected to the fluid path network and is configured to, in response to the accommodating chamber reaching the fluid guiding position, pierce the first pierceable structure 210 with the first end thereof and then insert into the reagent slot 20a to expose the reagent slot 20a so as to change an air pressure inside the reagent slot 20a. Each fluid guiding needle is constructed as a hollow needle, in fluid communication with a corresponding branch fluid passage of the plurality of branch fluid passages in one-to-one correspondence, and is configured to, in response to a situation where the accommodating chamber reaches the fluid guiding position, pierce the second pierceable structure 220 with the second end thereof and then insert into the guide slot 20b for fluid communication to the guide slot 20b so as to draw the fluid in the guide slot 20b.

As an example, more specifically, in a case that the plurality of fluid guiding needles are inserted into the guide slot 20b, a free end of each of the plurality of fluid guiding needles is higher than an inner wall at a bottom of the reagent slot 20a.

As an example, more specifically, each guide slot 20b is constructed as a slot having a circular cross-section with a first inner diameter, and each guide slot 20b is constructed as a slot having a circular cross-section with a second inner diameter smaller than the first inner diameter.

As an example, more specifically, the first pierceable structure is a metal foil; and the second pierceable structure is a flexible sealing element, such that a liquid-tight seal is maintained after being pierced by a corresponding fluid guiding needle of the plurality of fluid guiding needles.

The gene sequencing apparatus 5 includes the aforementioned chip processing device 1 and the aforementioned gene sequencer 4, thus possessing all the advantages of the aforementioned chip processing device 1, which will not be repeated here. Moreover, by providing the two aforementioned gene sequencers 4 arranged in mirror symmetry, two chips 3 with a minimum spacing may be simultaneously operated, a moving distance of switching chips 3 is greatly shortened, and a testing time interval is correspondingly shortened.

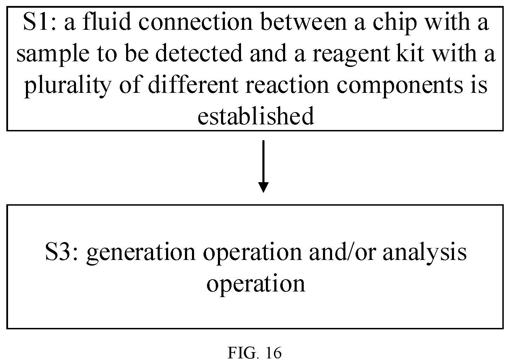

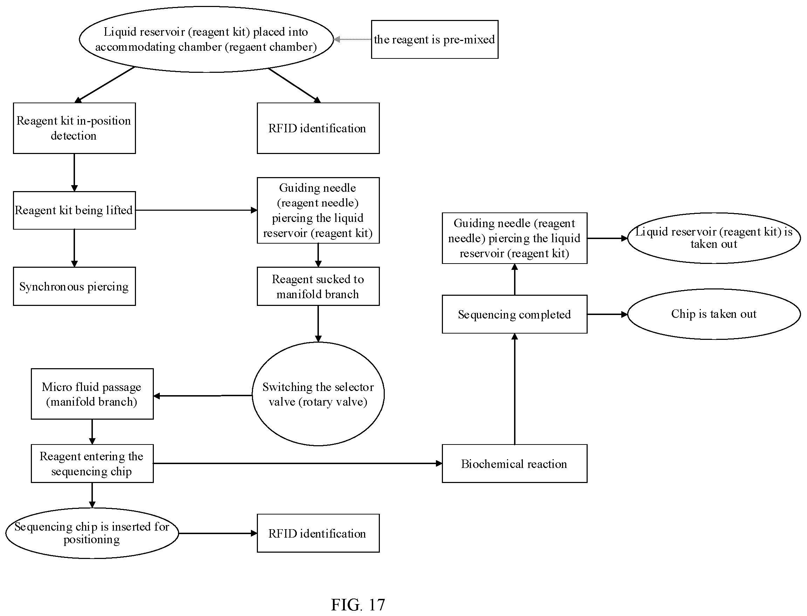

FIG. 16 shows a schematic flowchart of a biochemical detection method using the gene sequencer 4 shown in FIG. 14 according to an embodiment of the present disclosure. FIG. 17 schematically shows more detailed steps in the method of the flowchart shown in FIG. 16.

According to another aspect of embodiments of the present disclosure, based on the overall technical concept of embodiments of the present disclosure, for example, as shown in FIG. 16, there is further provided a biochemical detection method using the aforementioned gene sequencer 4. The gene sequencer includes: a chip carrying a sample for fluid detection; and a chip processing device, the chip processing device includes a substrate extending in a first direction, and a reagent kit platform and a chip platform assembled side-by-side and adjacently on the substrate in a second direction transverse to the first direction, a top plate of the chip platform on a side away from the substrate has a chip receiving area for accommodating the chip, the reagent kit platform is formed with a hollow accommodating chamber, and the reagent kit is received in the accommodating chamber and at least partially filled with the fluid inside. For example, the reagent kit has a first fluid transport structure located on a side of the reagent kit towards the chip platform in the second direction, and the chip platform has a second fluid transport structure located on a side of the chip platform towards the reagent kit platform in the second direction and configured to at least partially overlap and communicate with the first fluid transport structure in a case that the reagent kit platform is assembled with the chip platform. For example, the reagent kit is removably inserted into the accommodating chamber, and the fluid in the reagent kit is in fluid communication with the sample carried on the chip via the first fluid transport structure and the second fluid transport structure.

As an example, the biochemical detection method includes: S1: a fluid connection between a chip with a sample to be detected and a reagent kit with a plurality of different reaction components is established, the reaction components include at least one of a specimen generation component or a specimen analysis component; S2: a generation operation and/or an analysis operation is performed. The reagent kit and the chip are integrated into the chip processing device described in the above-mentioned embodiments, and the fluid in the reagent kit is in fluid communication with the chip via the first fluid transport structure and the second fluid transport structure separated from each other in the chip processing device.

Optionally, a specimen to be detected is generated on the chip in the generation operation, the generation operation includes flowing different specimen generation components into the chip and controlling reaction conditions of the chip to generate the specimen. The specimen of the chip is analyzed in the analysis operation, and the analysis operation includes flowing the specimen analysis component into the chip, and the specimen analysis component reacts with the specimen to provide a relevant detectable signal. The biochemical reaction may be a nucleic acid sequencing reaction or an antigen antibody binding reaction, and the specimen to be detected may be a nucleic acid sequencing library or a biological tissue slice, and the detectable signal is preferably an optical signal.

The first fluid transport structure is communicated with an external air pressure of the chip processing device to drive the fluid in the reagent kit to flow into the second flow transport structure; and the chip is selectively communicated with the fluid. For example, the second flow transport structure has a plurality of branch fluid passages as described above, and the selective communication between different branch fluid passages and the chip is controlled using the above-mentioned selector valve 126 integrated in the chip processing device.

In order to enable those skilled in the art to more clearly understand the present invention, more detailed steps of the method shown in FIG. 16 are shown below in conjunction with FIG. 17.

In a more detailed embodiment of the present disclosure, for example, step S1 of establishing a fluid connection between the chip and the reagent kit includes: the reagent kit (having a similar structure to the liquid reservoir 2 described in the above-mentioned embodiments) is inserted into the accommodating chamber 110 through an opened opening of the accommodating chamber 110, and whether the reagent kit is in position inside the accommodating chamber 110 is detected by using the aforementioned in-position detector 112 provided inside the accommodating chamber 110, and the reagent kit is kept in position by using the positioning bead 111b of the aforementioned positioning device 111 in the accommodating chamber 110 to press against the reagent kit under the elastic pushing action of the spring 111a.

In a specific embodiment, for example, the accommodating chamber is coupled to the substrate through a movable bracket (equivalent to the above-mentioned movable bracket 11a) to achieve movement in the chip processing device in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively. As an example, the chip processing device is configured to: in response to the accommodating chamber being lifted away from the substrate to the fluid guiding position, the first fluid transport structure is engaged with the second fluid transport structure for fluid communication; and in response to the accommodating chamber being lowered towards the substrate to the non-fluid guiding position, the first fluid transport structure is separated from the second fluid transport structure. Under this arrangement, the method further includes: after the reagent kit is inserted into the accommodating chamber in position, the accommodating chamber is lifted to the fluid guiding position, thereby triggering a subsequent fluid communication of the reagent kit to the carrier (having a similar structure to the top plate 120 in the above-mentioned embodiments) supporting the chip.

In a specific embodiment, for example, the top plate on a side of the chip platform away from the substrate has a flange protruding towards the reagent kit platform in the second direction, and the reagent kit platform is partially embedded and assembled between the flange of the top plate and the substrate. As an example, the first fluid transport structure includes a plurality of reagent slots 20a and a plurality of guide slots 20b arranged in one-to-one correspondence inside the reagent kit, and a communication channel 20c in fluid communication between a bottom of each guide slot 20b and a bottom of a corresponding reagent slot 20a. Each reagent slot 20a is at least partially filled with the fluid and has a first opening 21 open upward towards the flange and a first pierceable structure 210 covering the first opening 21, and each guide slot 20b has a second opening 22 open upward towards the flange and a second pierceable structure 220 covering the second opening 22. As an example, the second fluid transport structure further includes a fluid guiding component protruding, including: a plurality of membrane breaking needles and a plurality of fluid guiding needles, respectively protruding from a side of the top plate opposite to the chip receiving area towards the reagent kit platform, each membrane breaking needle has a first end aligned with a corresponding first pierceable structure 210, and each fluid guiding needle has a second end aligned with a corresponding second pierceable structure 220. Each membrane breaking needle is configured to, in response to the accommodating chamber reaching the fluid guiding position, pierce the first pierceable structure 210 with the first end thereof and then insert into the reagent slot 20a to expose the reagent slot 20a so as to change an air pressure inside the reagent slot 20a. Each fluid guiding needle is constructed as a hollow needle, in selective fluid communication with the sample carried on the chip, and is configured to, in response to a situation where the accommodating chamber reaches the fluid guiding position, pierce the second pierceable structure 220 with the second end thereof and then insert into the guide slot 20b for fluid communication to the guide slot 20b so as to draw the fluid in the guide slot 20b. Thus, the method further includes lowering of the accommodating chamber 110. Specifically, after the accommodating chamber is lifted to the fluid guiding position, the fluid communication between the first fluid transport structure and the second fluid transport structure is established by driving the corresponding first end of each membrane breaking needle to pierce the first pierceable structure 210 and driving the corresponding second end of each fluid guiding needle to pierce the second pierceable structure 220; and after the accommodating chamber is lowered and reset from the fluid guiding position to the non-fluid guiding position, the fluid communication between the first fluid transport structure and the second fluid transport structure is cut off by driving the corresponding first end of each membrane breaking needle to detach from the first pierceable structure 210 and driving the corresponding second end of each fluid guiding needle to detach from the second pierceable structure 220.

In a further embodiment, as an example, step S1 further includes: adjusting the adjustable thread pairs of the aforementioned first tilt adjustment mechanism 114 and the aforementioned second tilt adjustment mechanism 124 respectively to level the reagent kit platform 11 and the chip platform 12. Specifically, the reagent kit platform 11 is leveled and stably supported by adjusting a three-point support structure jointly defined by the first fix distance head and two adjustable first threaded pairs 1140 in the first tilt adjustment structure, and the chip platform 12 is leveled and stably supported by adjusting another three-point support structure jointly defined by the second fix distance head and two adjustable second threaded pairs 1240 in the second tilt adjustment structure.

In a further embodiment, as an example, step S1 further includes: using the aforementioned pressing device 113 formed at the top of the reagent kit platform 11, a further pivot of the pair of arms 1132 around the pin 1131 under the elastic force of the coupled pair of springs 111a is triggered by slightly pushing one of the pair of arms 1132 around the pin 1131, pressing the platen 1130 against the top of the accommodating chamber 110 and the liquid reservoir 2, thereby achieving further downward pressing of the accommodating chamber 110 together with the liquid reservoir 2 in a substantially vertical direction.

In a more specific embodiment of the present disclosure, for example, step S1 further includes: driving the accommodating chamber 110 to perform an upward movement from an initial low position by the aforementioned driving source via the guidance of each set of cross roller guide rails 130 in the at least one guide rail component 13.

In a further embodiment, as an example, step S1 further includes: stopping the driving source in response to a situation where the accommodating chamber 110 is lifted to an upper limit position by the aforementioned position limiting device 14.

In a more specific embodiment of the present disclosure, for example, step S1 further includes: using the fluid guiding needle in the aforementioned fluid guiding component 123 to pierce the liquid reservoir 2, and then using the aforementioned selector valve 126 (e.g. the rotary valve) to switch to fluid communication with the desired branch fluid passage 1252, thereby the desired branch fluid passage 1252 leading to the liquid reservoir 2 (e.g. the reagent kit) is in fluid communication with the sequencing chip 3 via the selector valve 126 and the single common fluid passage 1253.

In a more specific embodiment of the present disclosure, for example, step S1 further includes: placing the chip 3 (e.g., the gene sequencing chip 3) in the chip receiving area 121a formed on the carrier (the top plate 120) of the chip platform 12 of the chip processing device 1, and keeping the chip 3 in position in the chip receiving area 121a, for example, via pressing at a sidewall of the chip receiving area 121a and adsorption at a bottom of the chip receiving area 121a (e.g., achieved by adsorption force generated by an adsorption device such as a negative pressure adsorption device or a magnetic adsorption device).

In a more specific embodiment of the present disclosure, for example, step S1 includes: introducing the reagent fluid from the selector valve 126 and the single common fluid passage 1253 into the chip receiving area 121a accommodating the chip 3, achieving contact between the reagent and the specimen, and performing the above-mentioned analysis operation and/or generation operation.

In a more specific embodiment of the present disclosure, for example, the method further includes "removing the chip 3 from the chip processing device 1", for example, the chip 3 is taken out from the chip processing device 1 by disabling/canceling the adsorption force and subsequently removing the chip 3 from the chip receiving area 121a.

In a more specific embodiment of the present disclosure, for example, the method further includes: driving the accommodating chamber 110 to perform a downward motion until the accommodating chamber 110 returns to an initial low position by the aforementioned driving source via the guidance of each set of cross roller guide rails 130 in the at least one guide rail component 13.

In a more specific embodiment of the present disclosure, for example, the liquid reservoir 2 is released from the accommodating chamber 110 by first counter-pivoting the pressing device 113, and subsequently the liquid reservoir 2 is removed from the opened opening of the accommodating chamber 110.

The method of using the aforementioned gene sequencer 4 adopts the aforementioned chip processing device 1 and the aforementioned gene sequencer 4, thus possessing all the advantages of the aforementioned chip processing device 1, which will not be repeated here.

Therefore, the chip processing device, the gene sequencer, the gene sequencing apparatus, and the method of applying the gene sequencer disclosed in embodiments of the present disclosure have the following superior technical effects compared to the related art.

The chip processing device, the gene sequencer, the gene sequencing apparatus, and the biochemical detection method implemented in embodiments of the present disclosure, especially the chip processing device, may achieve an integrated ductless fluid transport structure through the above arrangements, which has a reduced fluid passage length, more reliable leveling and fastening effects and higher positioning accuracy for the reagent kit. Furthermore, the arrangement of sucking liquid from the top of the reagent kit in combination with the use of the injection pump may avoid the long stroke translating and lifting movements and also avoid the need to insert the reagent needle into the bottom of the reagent kit as in conventional operations. Accordingly, it is possible to improve the fixation, positioning, stroke, and other aspects of the fluid transport structure while enhancing the degree of integration and thereby improving the space utilization rate, and the design expectation is achieved with a more compact structure. Moreover, this compact structure minimizes the space occupation, and the simple construction and coupling relationship facilitate assembly and disassembly.

In the descriptions of the specification, the reference terms "an embodiment", "some embodiments", "example", "specific examples", "some examples" or the like means that the specific features, structures, materials, or characteristics described in conjunction with the embodiment or example is included in at least one embodiment or example of the present invention. In the specification, the schematic expressions of the above-described terms are not necessarily directed to the same embodiment or example. Moreover, the specific features, structures, materials, or characteristics described may be combined in any one or more embodiments or examples in an appropriate manner. In addition, those skilled in the art may incorporate and combine different embodiments or examples and the features of the different embodiments or examples described in the specification without contradicting each other.

Although the present disclosure has been described in combination with the accompanying drawings, the embodiments disclosed in the accompanying drawings are intended to illustrate the preferred embodiments of the present disclosure, and are not to be understood as limiting the present disclosure.

Some embodiments of the general concept of the present disclosure have been shown and illustrated. However, those skilled in the art will understand that these embodiments may be changed without departing from the principle and spirit of the present disclosure, and the scope of the present disclosure is defined by the claims and their equivalents.

## Claims

1. A chip processing device integrated with a reagent kit, comprising: a substrate extending in a first direction, and a reagent kit platform and a chip platform assembled side-by-side and adjacently on the substrate in a second direction transverse to the first direction, wherein a top plate of the chip platform on a side away from the substrate has a chip receiving area for accommodating a chip carrying a sample for fluid detection, the reagent kit platform is formed with a hollow accommodating chamber, and the reagent kit is received in the accommodating chamber,
wherein the reagent kit has a first fluid transport structure located inside the reagent kit on a side towards the chip platform in the second direction, the chip platform has a second fluid transport structure located on a side of the chip platform towards the reagent kit platform in the second direction and configured to at least partially overlap and communicate with the first fluid transport structure in a case that the reagent kit platform is assembled with the chip platform, and the first fluid transport structure is in fluid communication with the chip via the second fluid transport structure.

2. The chip processing device according to claim 1, wherein the accommodating chamber is coupled to the substrate in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively; and
wherein the chip processing device is configured to:
in response to the accommodating chamber being lifted away from the substrate to the fluid guiding position, the first fluid transport structure is engaged with the second fluid transport structure for fluid communication; and
in response to the accommodating chamber being lowered towards the substrate to the non-fluid guiding position, the first fluid transport structure is separated from the second fluid transport structure.

3. The chip processing device according to claim 2, wherein the top plate on a side of the chip platform away from the substrate has a flange protruding towards the reagent kit platform in the second direction, and the reagent kit platform is partially embedded and assembled between the flange of the top plate and the substrate.

4. The chip processing device according to claim 3, wherein the first fluid transport structure comprises a plurality of reagent slots and a plurality of guide slots arranged in one-to-one correspondence inside the reagent kit, and a communication channel in fluid communication between a bottom of each of the plurality of guide slots and a bottom of a corresponding reagent slot, each reagent slot is at least partially filled with a fluid and has a first opening open upward towards the flange and a first pierceable structure covering the first opening, and each guide slot has a second opening open upward towards the flange and a second pierceable structure covering the second opening.

5. The chip processing device according to claim 4, wherein the second fluid transport structure comprises a fluid supply device, and the fluid supply device comprises:
a fluid path network formed in the top plate of the chip platform and communicated between the first fluid transport structure and the chip, and
a selector valve installed to the top plate and in fluid communication with the fluid path network.

6. The chip processing device according to claim 5, wherein,
the selector valve comprises:
a valve seat, wherein the selector valve is fixed to the top plate via the valve seat; and
a valve body, extending from the valve seat in a direction away from the top plate, wherein the valve body is formed with a fluid inlet configured to guide the fluid to flow into an interior of the valve body and a fluid outlet configured to guide the fluid to flow outward from the interior of the valve body,
wherein the fluid path network comprises:
a plurality of fluid guiding needle ports located on a side of the top plate opposite to the chip receiving area;
a plurality of inlet ports spaced apart from the plurality of fluid guiding needle ports in one-to-one correspondence;
a plurality of branch fluid passages, each of the plurality of branch fluid passages is communicated between each fluid guiding needle port and a corresponding inlet port, and is configured to guide the fluid input from each of the plurality of fluid guiding needle port to the corresponding inlet port;
an outlet port spaced apart from the plurality of inlet ports and not in communication with the plurality of branch fluid passages; and
a common fluid passage communicated between the outlet port and the chip receiving area, the common fluid passage is configured to guide the fluid output from the outlet port to the chip receiving area, and
wherein the selector valve is arranged such that the fluid outlet is in fluid communication with the outlet port, and the fluid inlet is in fluid communication with at least one of the plurality of inlet ports, and the selector valve is configured to switch a selective communication between at least one of the plurality of inlet ports corresponding to the plurality of branch fluid passages and the outlet port via the fluid inlet and the fluid outlet.

7. The chip processing device according to claim 6, wherein the plurality of fluid guiding needle ports are penetratively formed in a protruding portion at an edge of the top plate and protruding towards a side of the top plate away from the chip receiving area.

8. The chip processing device according to claim 6, wherein the second fluid transport structure further comprises a fluid guiding component protruding from the flange towards the reagent kit platform and being communicated to the chip receiving area, and the fluid guiding component comprises: a plurality of membrane breaking needles and a plurality of fluid guiding needles respectively protruding from a side of the top plate opposite to the chip receiving area towards the reagent kit platform, each of the plurality of membrane breaking needles has a first end aligned with a corresponding first pierceable structure, and each of the plurality of fluid guiding needles has a second end aligned with a corresponding second pierceable structure,
wherein each membrane breaking needle is not connected to the fluid path network and is configured to, in response to the accommodating chamber reaching the fluid guiding position, pierce the first pierceable structure with the first end and then insert into the reagent slot to expose the reagent slot so as to change an air pressure inside the reagent slot; and
wherein each fluid guiding needle is configured as a hollow needle, in fluid communication with a corresponding branch fluid passage of the plurality of branch fluid passages in one-to-one correspondence, and is configured to, in response to a situation where the accommodating chamber reaches the fluid guiding position, pierce the second pierceable structure with the second end and then insert into the guide slot for fluid communication to the guide slot so as to draw the fluid in the guide slot.

9. The chip processing device according to claim 8, wherein in a case that the plurality of fluid guiding needles are inserted into the guide slots, a free end of each of the plurality of fluid guiding needles is higher than an inner wall at a bottom of the reagent slot.

10. The chip processing device according to claim 9, wherein each guide slot has a slot having a circular cross-section with a first inner diameter, and each guide slot has a slot having a circular cross-section with a second inner diameter smaller than the first inner diameter.

11. The chip processing device according to claim 8, wherein the first pierceable structure is a metal foil; and
wherein the second pierceable structure is a flexible sealing element, such that a liquid-tight seal is maintained after being pierced by a corresponding fluid guiding needle of the plurality of fluid guiding needles.

12. The chip processing device according to claim 8, wherein the plurality of fluid guiding needles are spaced apart from each other in a straight line, and the plurality of membrane breaking needles are spaced apart from each other in a straight line; and
wherein the plurality of fluid guiding needles and the plurality of membrane breaking needles are arranged parallel to each other.

13. The chip processing device according to claim 12, wherein a corresponding membrane breaking needle is provided aside each fluid guiding needle in one-to-one correspondence, each fluid guiding needle is in pair with the corresponding single membrane breaking needle, and each fluid guiding needle and the corresponding single membrane breaking needle are arranged adjacent to and spaced apart from each other.

14. The chip processing device according to claim 8, wherein each fluid guiding needle comprises a hollow and elongated straight tubular needle body, and a through terminal with a tapered longitudinal cross-section.

15. The chip processing device according to claim 8, wherein the plurality of fluid guiding needles are installed to the plurality of fluid guiding needle ports in a threaded connection manner.

16. The chip processing device according to claim 8, wherein the fluid guiding component further comprises: at least two guide pins protruding outward from a side of the top plate opposite to the chip receiving area and arranged spaced apart from each other in a straight line, and configured to be in positive fit with alignment features on the reagent kit.

17. The chip processing device according to claim 6, wherein the plurality of fluid guiding needle ports are aligned in a straight line.

18. The chip processing device according to claim 17, wherein the plurality of fluid guiding needle ports are spaced from each other by a uniform spacing.

19. The chip processing device according to claim 6, wherein the selector valve is a rotary valve configured to be rotatable about an axis of the rotary valve to switch a selective communication between at least one of the plurality of inlet ports corresponding to the plurality of branch fluid passages and the outlet port via the fluid inlet and the fluid outlet.

20. The chip processing device according to claim 19, wherein an axial direction of the inlet port and an axial direction of the outlet port are parallel to the axis, and the inlet port is offset from the axis, and the outlet port is coaxial with the axis.

21. The chip processing device according to claim 20, wherein the selector valve is installed to a back side of the top plate away from the chip receiving area via the valve seat, and
wherein the plurality of inlet ports are arranged in a circular shape around the axis, the plurality of inlet ports and the outlet port are open to the back side of the top plate, and at least one of the plurality of inlet ports is selectively communicated to the fluid inlet of the valve body, the outlet port is communicated to the fluid outlet of the valve body, and a center-to-center distance between each of the plurality of inlet ports and the outlet port is equal.

22. The chip processing device according to claim 19, wherein the plurality of branch fluid passages diverge radially around the rotary valve to communicate with the plurality of fluid guiding needle ports in one-to-one correspondence, respectively.

23. The chip processing device according to claim 6, wherein the top plate comprises: a top layer, wherein the chip receiving area is formed in the top layer; and a support layer stacking with the top layer, and the support layer is located between the top layer and the selector valve, and
wherein the plurality of branch fluid passages and the common fluid passage are formed on a side of the support layer towards the top layer, and the plurality of inlet ports, the outlet port, and the plurality of fluid guiding needle ports extend to penetrate the support layer.

24. The chip processing device according to claim 23, wherein the chip receiving area of the top layer is formed therein a through accommodating hole, and a recess recessing from a side of the top layer away from the support layer and arranged around the accommodating hole.

25. The chip processing device according to claim 24, wherein the top plate further comprises a chip adsorption component partially fixed in the accommodating hole and protruding away from the support layer from the accommodating hole, the chip adsorption component is configured to accommodate and adsorb the chip, and the chip adsorption comprises:
a pipe joint in communication with a negative pressure source;
an adsorption table partially arranged in the accommodating hole, wherein an edge of a top surface of the adsorption table protrudes outward to define an adsorption slot in the top layer recessing towards the support layer and located between the edge and the top surface, the adsorption slot is configured as a closed slot extending along the edge and in a closed loop form, and a shape and a size of the adsorption slot are determined to be suitable for surrounding and securing the edge of the chip; and
a negative pressure channel penetratively formed in the adsorption table and communicated between the pipe joint and the adsorption slot.

26. The chip processing device according to claim 24, wherein the top layer is formed with a discharge port and a supply port penetrating the top layer and extending to the recess, the supply port is in fluid communication with the outlet port of the valve body via the common fluid passage, and the discharge port is spaced apart from the supply port.

27. The chip processing device according to claim 26, wherein the common fluid passage is coupled between the outlet port and the supply port in a straight line.

28. The chip processing device according to claim 27, wherein the plurality of branch fluid passages are arranged not to cross each other and to avoid the common fluid passage.

29. The chip processing device according to claim 26, wherein end portions of the discharge port and the supply port respectively extend to the recess; and
wherein in a case that the chip is received and adsorbed on the adsorption table, the adsorption table and the chip jointly define a first liquid-tight sealing surface, and the chip is in a sealed fluid communication with the supply port and the discharge port respectively at the first liquid-tight sealing surface.

30. The chip processing device according to claim 26, wherein the support layer is formed with a discharge channel penetrating the support layer and in fluid communication from the discharge port to an outside of the top plate.

31. The chip processing device according to claim 23, wherein the plurality of inlet ports and the outlet port extend to penetrate the support layer to the back side of the top plate, and the back side and the valve seat jointly define a second liquid-tight sealing surface, and the plurality of inlet ports and the outlet port respectively form a sealed fluid communication with the fluid inlet and the fluid outlet of the valve body at the second liquid-tight sealing surface.

32. The chip processing device according to claim 8, wherein the chip processing device further comprises a driving component inside, at least one guide rail component coupled between the accommodating chamber and the substrate, the accommodating chamber is movable in a direction orthogonal to the substrate via a transmission of at least one guide rail component by the driving component, so that the fluid guiding component is inserted into and fluidly communicated to the reagent kit.

33. The chip processing device according to claim 32, wherein the driving component comprises: a driving source comprising one of a stepper motor and a piezoelectric driver; and a lead screw in transmission coupling between the driving source and at least one guide rail component.

34. The chip processing device according to claim 33, wherein each guide rail component comprises two spaced sets of cross roller guide rails, each set of cross roller guide rails comprises a fixed rail fixed to the chip platform, a movable rail coupled to the accommodating chamber, and a plurality of rolling elements held between the fixed rail and the movable rail.

35. The chip processing device according to claim 34, wherein in each set of cross roller guide rails, the fixed rail is fixed to the chip platform in a manner orthogonal to the substrate, and the movable rail is fixed to the accommodating chamber in a manner orthogonal to the substrate.

36. The chip processing device according to claim 35, wherein the lead screw is coupled with the respective movable rail of the two sets of cross roller guide rails in at least one guide rail component.

37. The chip processing device according to claim 1, further comprising a positioning device, wherein the positioning device comprises:
a groove concavely formed in a top-side inner wall of the accommodating chamber;
an elastic component arranged in the groove, and
a positioning bead arranged at an end of the elastic component towards the substrate and configured to:
in response to a situation where the reagent kit does not reach the positioning bead within the accommodating chamber, the elastic component is in an initial state not subjected to a force applied by the reagent kit, and the positioning bead protrudes at least partially towards the substrate from the top-side inner wall of the accommodating chamber; and
in response to a situation where the reagent kit is inserted into the accommodating chamber and the positioning bead is pressed, the elastic component is pushed towards the groove via the positioning bead, thereby causing the elastic component to retract at least partially into the groove.

38. The chip processing device according to claim 37, wherein the reagent kit has a protruding portion protruding towards the top-side inner wall of the accommodating chamber, and a positioning groove recessing from the protruding portion, and
in response to a situation where the reagent kit is inserted into the accommodating chamber and the positioning bead is pressed, the positioning bead is clamped between the positioning groove and the elastic component.

39. The chip processing device according to claim 1 or 37, further comprising an in-position detector inside the accommodating chamber, wherein the in-position detector comprises an optocoupler component and a shielding member, the optocoupler component comprises an infrared emitter and an infrared receiver and is configured to determine that the reagent kit is in position inside the accommodating chamber, in response to the shielding member being displaced or deformed by a force applied by the reagent kit and reception of infrared rays from the infrared emitter by the infrared receiver being blocked when the reagent kit is inserted in position inside the accommodating chamber.

40. The chip processing device according to claim 1, further comprising a pressing device formed at a top of the reagent kit platform, wherein the top of the reagent kit platform is formed with a through opening to at least partially expose the reagent kit inserted into the accommodating chamber, and the pressing device comprises:
a platen, wherein one side of the platen is fixed to the reagent kit platform;
a pin penetrating a free side of the platen opposite to the one side;
a pair of arms, wherein one end of each of the pair of arms is rotatably coupled to a corresponding end of two ends of the pin; and
a pair of elastic members, wherein each elastic member is elastically coupled between a corresponding arm of the pair of arms and the reagent kit platform.

41. The chip processing device according to claim 1, further comprising a first tilt adjustment mechanism arranged between a bottom of the reagent kit platform and the substrate, wherein the first tilt adjustment mechanism comprises a first fix distance head and two first thread pairs being rotatable and adjustable relative to the substrate that are arranged in a non-straight line.

42. The chip processing device according to claim 1, further comprising a second tilt adjustment mechanism arranged between a bottom of the chip platform and the substrate, wherein the second tilt adjustment mechanism comprises a second fix distance head and two second threaded pairs being rotatable and adjustable relative to the substrate that are arranged in a non-straight line.

43. The chip processing device according to claim 34, further comprising a position limiting device, wherein the position limiting device comprises:
an induction sheet arranged on a corresponding movable rail of at least one set of cross roller guide rails in at least one guide rail component; and
an upper position limiting optocoupler and a lower position limiting optocoupler respectively arranged at positions corresponding to upper and lower ends of a stroke of the corresponding movable rail at two ends of the corresponding fixed rail of the at least one set of cross roller guide rails, and respectively configured to stop the driving source in response to determining that the movable rail is lifted to to an upper limit position by detecting that the upper position limiting optocoupler is blocked by the induction sheet, and to stop the driving source in response to determining that the movable rail is lowered to a lower limit position by detecting that the lower position limiting optocoupler is blocked by the induction sheet.

44. The chip processing device according to any one of claims 8 to 16, further comprising a power component in fluid communication with the fluid path network, wherein the power component is configured to drive the fluid to flow through the fluid path network towards the supply port.

45. The chip processing device according to claim 44, wherein the power component comprises a pump in communication with the chip receiving area, and the pump is configured to provide a negative pressure to the chip receiving area.

46. The chip processing device according to claim 44, further comprising a temperature control component arranged on a side of the top plate opposite to the chip receiving area and configured to regulate a temperature of the fluid supplied from the fluid guiding component to the chip receiving area.

47. The chip processing device according to claim 46, wherein the temperature control component comprises a first temperature adjustment device arranged adjacent to the selector valve on a same side of the top plate, and
wherein a fluid guiding device further comprises an adapter bracket fixed to a side of the top plate opposite to the chip receiving area and configured as a form of a frame, and the adapter bracket is formed with two concave cavities arranged side-by-side and recessing from opposite sides of the adapter bracket away from the top plate and towards the top plate, respectively, so as to respectively accommodate and fix the selector valve and the first temperature adjustment device inside.

48. The chip processing device according to claim 47, wherein the first temperature adjustment device comprises:
a heat dissipation component accommodated and installed in one of the two concave cavities of the adapter bracket recessing from a side towards the top plate, wherein the heat dissipation component comprises: at least one of an active heat dissipation component and a passive heat dissipation component; and
a temperature control module installed to the heat dissipation component and configured to cut off the power component when a temperature at the heat dissipation component exceeds a threshold temperature.

49. The chip processing device according to claim 47, wherein the temperature control component further comprises a heat conducting member inserted between the top plate and the first temperature adjustment device.

50. The chip processing device according to claim 49, wherein the heat conducting member comprises a phase change material.

51. The chip processing device according to claim 48, wherein the active heat dissipation component comprises a thermoelectric cooler; or
the passive heat dissipation component comprises one of: a single heat sink or a heat sink array.

52. The chip processing device according to claim 47, wherein the temperature control component further comprises a second temperature adjustment device fixed to a side of the adapter bracket away from the top plate and arranged side-by-side with the selector valve, and the second temperature adjustment device is aligned with the first temperature adjustment device.

53. The chip processing device according to claim 52, wherein the second temperature adjustment device comprises a fan, and the fan comprises:
a hollow first housing defining a cavity inside for air flow, wherein the first housing is arranged such that the cavity is aligned with the first temperature adjustment device and open towards the first temperature adjustment device, so as to fluidly communicate the cavity between the first temperature adjustment device and an outside of the fluid guiding device; and
a fan component accommodated inside the first housing, wherein the fan component comprises:
a second housing configured as a hollow cylindrical body fixedly sleeved inside the first housing;
a rotating shaft rotatably installed inside the second housing; and
a plurality of fan blades coaxially fixed to the rotating shaft inside the second housing and rotatable with the rotating shaft relative to the second housing.

54. The chip processing device according to claim 53, wherein the adapter bracket is further formed with a gas channel penetrating an inside of the adapter bracket and open at opposite ends respectively towards cavities of the first temperature adjustment device and the second temperature adjustment device, and the cavities are communicated to the first temperature adjustment device via the gas channel.

55. The chip processing device according to claim 53, wherein the second temperature adjustment device further comprises a vibration reduction device, and the vibration reduction device comprises:
a first level vibration reduction structure configured as a gasket inserted between the adapter bracket and the first housing of the fan; and
a second level vibration reduction structure arranged inside the first housing of the fan between the second housing of the fan component and the first housing and, wherein the second level vibration reduction structure comprises a plurality of vibration reduction members respectively snap-fitted to an inner wall of the first housing and spaced apart from each other, and the second housing of the fan component is coupled to the first housing via the plurality of vibration reduction members.

56. The chip processing device according to claim 55, wherein at least one of the first level vibration reduction structure and the second level vibration reduction structure is a vibration compensation device, and the vibration compensation device is an elastic member or a damping member.

57. The chip processing device according to claim 55, wherein the first housing of the fan is fixed to the adapter bracket via a threaded member of a flexible vibration absorbing material, and the first level vibration reduction structure is pressed between the adapter bracket and the first housing via the threaded member.

58. The chip processing device according to claim 47 or 57, wherein the selector valve is fixed to the top plate via a threaded connection at the valve seat, and the selector valve is coupled to the adapter bracket via an adjustable abutting device, and the adjustable abutting device comprises:
a plurality of fix distance screws respectively threadably penetrating the adapter bracket and abutting against the valve body of the selector valve; and
a plurality of springs elastically abutting against the plurality of fix distance screws towards the top plate in one-to-one correspondence, respectively.

59. A gene sequencer, comprising:
a chip carrying a sample for fluid detection; and
a chip processing device comprising a substrate extending in a first direction, and a reagent kit platform and a chip platform assembled side-by-side and adjacently on the substrate in a second direction transverse to the first direction, wherein a top plate of the chip platform on a side away from the substrate has a chip receiving area for accommodating the chip, the reagent kit platform is formed with a hollow accommodating chamber, and the reagent kit is received in the accommodating chamber and at least partially filled with a fluid inside,
wherein the reagent kit has a first fluid transport structure located inside the reagent kit on a side towards the chip platform in the second direction, the chip platform has a second fluid transport structure located on a side of the chip platform towards the reagent kit platform in the second direction and configured to at least partially overlap and communicate with the first fluid transport structure in a case that the reagent kit platform is assembled with the chip platform; and
wherein the reagent kit is removably inserted into the accommodating chamber, and the fluid in the reagent kit is in fluid communication with the sample carried on the chip via the first fluid transport structure and the second fluid transport structure.

60. The gene sequencer according to claim 59, wherein the accommodating chamber is coupled to the substrate in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively; and
wherein the chip processing device is configured to:
in response to the accommodating chamber being lifted away from the substrate to the fluid guiding position, the first fluid transport structure is engaged with the second fluid transport structure for fluid communication; and
in response to the accommodating chamber being lowered towards the substrate to the non-fluid guiding position, the first fluid transport structure is separated from the second fluid transport structure.

61. The gene sequencer according to claim 60, wherein the top plate on a side of the chip platform away from the substrate has a flange protruding towards the reagent kit platform in the second direction, and the reagent kit platform is partially embedded and assembled between the flange of the top plate and the substrate.

62. The gene sequencer according to claim 61, wherein the first fluid transport structure comprises a plurality of reagent slots and a plurality of guide slots arranged in one-to-one correspondence inside the reagent kit, and a communication channel in fluid communication between a bottom of each guide slot and a bottom of a corresponding reagent slot, each reagent slot is at least partially filled with the fluid and has a first opening open upward towards the flange and a first pierceable structure covering the first opening, and each guide slot has a second opening open upward towards the flange and a second pierceable structure covering the second opening.

63. The gene sequencer according to claim 62, wherein the second fluid transport structure comprises a fluid supply device, and the fluid supply device comprises:
a fluid path network formed in the top plate of the chip platform and communicated between the first fluid transport structure and the chip, and
a selector valve installed to the top plate and in fluid communication with the fluid path network.

64. The gene sequencer according to claim 63, wherein,
the selector valve comprises:
a valve seat, wherein the selector valve is fixed to the top plate via the valve seat; and
a valve body, extending from the valve seat in a direction away from the top plate, wherein the valve body is formed with a fluid inlet configured to guide the fluid to flow into an interior of the valve body and a fluid outlet configured to guide the fluid to flow outward from the interior of the valve body,
wherein the fluid path network comprises:
a plurality of fluid guiding needle ports located on a side of the top plate opposite to the chip receiving area;
a plurality of inlet ports spaced apart from the plurality of fluid guiding needle ports in one-to-one correspondence;
a plurality of branch fluid passages, each of the plurality of branch fluid passages is communicated between each fluid guiding needle port and a corresponding inlet port, and is configured to guide the fluid input from each of the plurality of fluid guiding needle port to the corresponding inlet port;
an outlet port spaced apart from the plurality of inlet ports and not in communication with the plurality of branch fluid passages; and
a common fluid passage communicated between the outlet port and the chip receiving area, the common fluid passage is configured to guide the fluid output from the outlet port to the chip receiving area, and
wherein the selector valve is arranged such that the fluid outlet is in fluid communication with the outlet port, and the fluid inlet is in fluid communication with at least one of the plurality of inlet ports, and the selector valve is configured to switch a selective communication between at least one of the plurality of inlet ports corresponding to the plurality of branch fluid passages and the outlet port via the fluid inlet and the fluid outlet.

65. The gene sequencer according to claim 64, wherein the plurality of fluid guiding needle ports are penetratively formed in a protruding portion at an edge of the top plate and protruding towards a side of the top plate away from the chip receiving area.

66. The gene sequencer according to claim 64, wherein the second fluid transport structure further comprises a fluid guiding component protruding from the flange towards the reagent kit platform and being communicated to the chip receiving area, and the fluid guiding component comprises: a plurality of membrane breaking needles and a plurality of fluid guiding needles respectively protruding from a side of the top plate opposite to the chip receiving area towards the reagent kit platform, each of the plurality of membrane breaking needles has a first end aligned with a corresponding first pierceable structure, and each of the plurality of fluid guiding needles has a second end aligned with a corresponding second pierceable structure,
wherein each membrane breaking needle is not connected to the fluid path network and is configured to, in response to the accommodating chamber reaching the fluid guiding position, pierce the first pierceable structure with the first end and then insert into the reagent slot to expose the reagent slot so as to change an air pressure inside the reagent slot; and
wherein each fluid guiding needle is configured as a hollow needle, in fluid communication with a corresponding branch fluid passage of the plurality of branch fluid passages in one-to-one correspondence, and is configured to, in response to a situation where the accommodating chamber reaches the fluid guiding position, pierce the second pierceable structure with the second end and then insert into the guide slot for fluid communication to the guide slot so as to draw the fluid in the guide slot.

67. The gene sequencer according to claim 66, wherein in a case that the plurality of fluid guiding needles are inserted into the guide slots, a free end of each of the plurality of fluid guiding needles is higher than an inner wall at a bottom of the reagent slot.

68. The gene sequencer according to claim 67, wherein each guide slot has a slot having a circular cross-section with a first inner diameter, and each guide slot has a slot having a circular cross-section with a second inner diameter smaller than the first inner diameter.

69. The gene sequencer according to claim 66, wherein the first pierceable structure is a metal foil; and
wherein the second pierceable structure is a flexible sealing element, such that a liquid-tight seal is maintained after being pierced by a corresponding fluid guiding needle of the plurality of fluid guiding needles.

70. A gene sequencing apparatus, comprising:
a chip carrying a sample for fluid detection; and
at least two chip processing devices, wherein each of the at least two chip processing devices comprises a substrate extending in a first direction, and a reagent kit platform and a chip platform assembled side-by-side and adjacently on the substrate in a second direction transverse to the first direction, wherein a top plate of the chip platform on a side away from the substrate has a chip receiving area for accommodating the chip, the reagent kit platform is formed with a hollow accommodating chamber, and the reagent kit is received in the accommodating chamber and at least partially filled with a fluid inside,
wherein the reagent kit has a first fluid transport structure located inside the reagent kit on a side towards the chip platform in the second direction, the chip platform has a second fluid transport structure located on a side of the chip platform towards the reagent kit platform in the second direction and configured to at least partially overlap and communicate with the first fluid transport structure in a case that the reagent kit platform is assembled with the chip platform; and
wherein the reagent kit is removably inserted into the accommodating chamber, and the fluid in the reagent kit is in fluid communication with the sample carried on the chip via the first fluid transport structure and the second fluid transport structure; and
wherein the at least two chip processing devices are arranged symmetrically with respect to each other, and the respective chip platforms are arranged adjacent to each other.

71. The gene sequencing apparatus according to claim 70, wherein the at least two chip processing devices comprise at least one pair of chip processing devices arranged in mirror symmetry with the respective chip platforms arranged adjacent to each other.

72. The gene sequencing apparatus according to claim 70, wherein the accommodating chamber is coupled to the substrate in a linearly movable manner within a range between a highest fluid guiding position and a non-fluid guiding position lower than the fluid guiding position, and the fluid guiding position and the non-fluid guiding position correspond to a state of fluid communication between the first fluid transport structure and the second fluid transport structure, and a state of non-fluid communication between the first fluid transport structure and the second fluid transport structure, respectively; and
wherein the chip processing device is configured to:
in response to the accommodating chamber being lifted away from the substrate to the fluid guiding position, the first fluid transport structure is engaged with the second fluid transport structure for fluid communication; and
in response to the accommodating chamber being lowered towards the substrate to the non-fluid guiding position, the first fluid transport structure is separated from the second fluid transport structure.

73. The gene sequencing apparatus according to claim 72, wherein the top plate on a side of the chip platform away from the substrate has a flange protruding towards the reagent kit platform in the second direction, and the reagent kit platform is partially embedded and assembled between the flange of the top plate and the substrate.

74. The gene sequencing apparatus according to claim 73, wherein the first fluid transport structure comprises a plurality of reagent slots and a plurality of guide slots arranged in one-to-one correspondence inside the reagent kit, and a communication channel in fluid communication between a bottom of each guide slot and a bottom of a corresponding reagent slot, each reagent slot is at least partially filled with the fluid and has a first opening open upward towards the flange and a first pierceable structure covering the first opening, and each guide slot has a second opening open upward towards the flange and a second pierceable structure covering the second opening.

75. The gene sequencing apparatus according to claim 74, wherein the second fluid transport structure comprises a fluid supply device, and the fluid supply device comprises:
a fluid path network formed in the top plate of the chip platform and communicated between the first fluid transport structure and the chip, and
a selector valve installed to the top plate and in fluid communication with the fluid path network.

76. The gene sequencing apparatus according to claim 75, wherein,
the selector valve comprises:
a valve seat, wherein the selector valve is fixed to the top plate via the valve seat; and
a valve body, extending from the valve seat in a direction away from the top plate, wherein the valve body is formed with a fluid inlet configured to guide the fluid to flow into an interior of the valve body and a fluid outlet configured to guide the fluid to flow outward from the interior of the valve body,
wherein the fluid path network comprises:
a plurality of fluid guiding needle ports located on a side of the top plate opposite to the chip receiving area;
a plurality of inlet ports spaced apart from the plurality of fluid guiding needle ports in one-to-one correspondence;
a plurality of branch fluid passages, each of the plurality of branch fluid passages is communicated between each fluid guiding needle port and a corresponding inlet port, and is configured to guide the fluid input from each of the plurality of fluid guiding needle port to the corresponding inlet port;
an outlet port spaced apart from the plurality of inlet ports and not in communication with the plurality of branch fluid passages; and
a common fluid passage communicated between the outlet port and the chip receiving area, the common fluid passage is configured to guide the fluid output from the outlet port to the chip receiving area, and
wherein the selector valve is arranged such that the fluid outlet is in fluid communication with the outlet port, and the fluid inlet is in fluid communication with at least one of the plurality of inlet ports, and the selector valve is configured to switch a selective communication between at least one of the plurality of inlet ports corresponding to the plurality of branch fluid passages and the outlet port via the fluid inlet and the fluid outlet.

77. The gene sequencing apparatus according to claim 76, wherein the plurality of fluid guiding needle ports are penetratively formed in a protruding portion at an edge of the top plate and protruding towards a side of the top plate away from the chip receiving area.

78. The gene sequencing apparatus according to claim 76, wherein the second fluid transport structure further comprises a fluid guiding component protruding from the flange towards the reagent kit platform and being communicated to the chip receiving area, and the fluid guiding component comprises: a plurality of membrane breaking needles and a plurality of fluid guiding needles respectively protruding from a side of the top plate opposite to the chip receiving area towards the reagent kit platform, each of the plurality of membrane breaking needles has a first end aligned with a corresponding first pierceable structure, and each of the plurality of fluid guiding needles has a second end aligned with a corresponding second pierceable structure,
wherein each membrane breaking needle is not connected to the fluid path network and is configured to, in response to the accommodating chamber reaching the fluid guiding position, pierce the first pierceable structure with the first end and then insert into the reagent slot to expose the reagent slot so as to change an air pressure inside the reagent slot; and
wherein each fluid guiding needle is configured as a hollow needle, in fluid communication with a corresponding branch fluid passage of the plurality of branch fluid passages in one-to-one correspondence, and is configured to, in response to a situation where the accommodating chamber reaches the fluid guiding position, pierce the second pierceable structure with the second end and then insert into the guide slot for fluid communication to the guide slot so as to draw the fluid in the guide slot.

79. The gene sequencing apparatus according to claim 78, wherein in a case that the plurality of fluid guiding needles are inserted into the guide slots, a free end of each of the plurality of fluid guiding needles is higher than an inner wall at a bottom of the reagent slot.

80. The gene sequencing apparatus according to claim 79, wherein each guide slot has a slot having a circular cross-section with a first inner diameter, and each guide slot has a slot having a circular cross-section with a second inner diameter smaller than the first inner diameter.

81. The gene sequencing apparatus according to claim 78, wherein the first pierceable structure is a metal foil; and
wherein the second pierceable structure is a flexible sealing element, such that a liquid-tight seal is maintained after being pierced by a corresponding fluid guiding needle of the plurality of fluid guiding needles.

82. A method of performing biochemical detection, comprising:
establishing a fluid connection between a chip with a sample for fluid detection and a reagent kit with a plurality of different reaction components, wherein the reaction components comprise at least one of a specimen generation component or a specimen analysis component;
optionally, generating a specimen on the chip in a generation operation, wherein the generation operation comprises flowing different specimen generation components into the chip and controlling reaction conditions of the chip to generate the specimen; and
analyzing the specimen of the chip in an analysis operation, wherein the analysis operation comprises flowing the specimen analysis component into the chip, and the specimen analysis component reacts with the specimen to provide a relevant detectable signal,
wherein a reagent kit and a chip are integrated into a chip processing device, and a fluid in the reagent kit is in fluid communication with the chip via a first fluid transport structure and a second fluid transport structure separated from each other in the chip processing device.

83. The method according to claim 82, wherein the first fluid transport structure is integrated into the reagent kit, the second fluid transport structure is integrated into a carrier supporting the chip, and the carrier is integrated into the chip processing device.

84. The method according to claim 83, wherein the reagent kit moves relative to the carrier through a movable bracket integrated into the chip processing device so as to achieve a communication between the first fluid transport structure and the second fluid transport structure.

85. The method according to claim 82, wherein the biochemical reaction is a nucleic acid sequencing reaction, and the specimen to be detected is a nucleic acid sequencing library.

86. The method according to claim 82, wherein the specimen to be detected is a tissue specimen, and the biochemical reaction is a specific binding reaction.

87. The method according to claim 82, wherein the detectable signal is an optical signal.

88. The method according to claim 84, wherein the communication between the first fluid transport structure and the second fluid transport structure comprises: the first fluid transport structure is communicated with an external air pressure of the chip processing device to drive the fluid in the reagent kit to flow into the second flow transport structure; and the chip is selectively communicated with the fluid in the second flow transport structure.

89. The method according to claim 88, wherein the second flow transport structure has a plurality of branch fluid passages, and the selective communication between the chip and the fluid comprises: controlling selective communications between different branch fluid passages and the chip using a selector valve integrated in the chip processing device.
